(19) Europäisches Patentamt
European Patent Office
Office européen des brevets

(11) EP 0 947 583 A2

(12) **EUROPÄISCHE PATENTANMELDUNG**

(43) Veröffentlichungstag:
**06.10.1999 Patentblatt 1999/40**

(51) Int. Cl.$^6$: **C12N 15/12**, C07K 14/47

(21) Anmeldenummer: **99101554.6**

(22) Anmeldetag: **19.07.1994**

(84) Benannte Vertragsstaaten:
**AT BE CH DE DK ES FR GB GR IE IT LI LU NL PT SE**

(30) Priorität: **20.07.1993 DE 4324247**

(62) Dokumentnummer(n) der früheren Anmeldung(en) nach Art. 76 EPÜ:
**94925364.5 / 0 710 248**

(71) Anmelder: **Roche Diagnostics GmbH**
**68305 Mannheim (DE)**

(72) Erfinder:
**Die Erfindernennung liegt noch nicht vor**

Bemerkungen:
Diese Anmeldung ist am 01 - 02 - 1999 als Teilanmeldung zu der unter INID-Kode 62 erwähnten Anmeldung eingereicht worden.

(54) **Expressionsaktivierende Nukleinsäurefragmente des Melanom-inhibierenden Proteins (MIA)**

(57) Expressionsaktivierende Nukleinsäurefragmente aus dem 5'-untranslatierten Bereich von SEQ ID NO:3 sind zur Herstellung von viralen oder nicht-viralen Vektoren für die Gentherapie geeignet.

**Fig. 6**

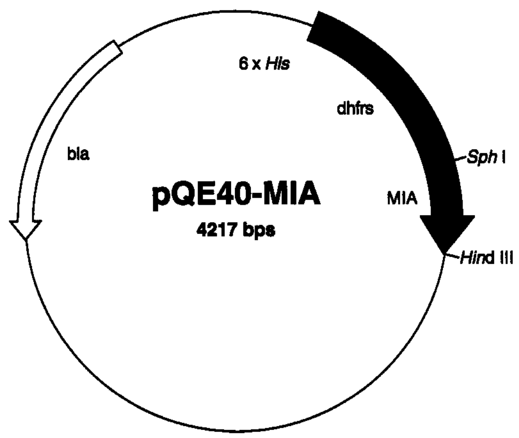




Printed by Xerox (UK) Business Services
2.16.7/3.6

**Beschreibung**

Die Erfindung betrifft ein Melanom-inhibierendes Protein (MIA), eine dafür codierende Nukleinsäure, ein Verfahren zur Gewinnung und zum Nachweis dieses Proteins sowie dessen Verwendung zur Herstellung eines Therapeutikums.

Die Regulation des Zellwachstums wird sowohl durch positiv als auch negativ wirkende Faktoren kontrolliert. Zu den positiv wirkenden Faktoren zählen die bekannten Wachstumsfaktoren wie z. B. epidermal growth factor (EGF), platelet derived growth factor (PDGF), Insulin und Somatomedine. Zu den negativ, d. h. inhibitorisch wirkenden Faktoren gehören neben dem TGF-β, der sowohl als Wachstumsstimulator als auch als Wachstumsinhibitor wirken kann (Roberts et al., Proc. Natl. Acad. Sci. 82 (1985), 119 - 123) die endogenen, tumorinhibierenden Faktoren aus Dickdarmkarzinomzellen (Levine et al, Cancer Research 45 (1985), 2248 - 2254), Melanomen (Bogdahn et al., Cancer Research 49 (1989), 5358 - 5363) sowie aus gesunden epithelialen Zellen von Brustdrüsen der Ratte (Ethier et al., J. Cell. Phys. 142 (1990), 15 - 20).

Durch Störung dieses Regulationssystems, wie z. B. durch Überproduktion von positiv wirkenden Wachstumsfaktoren oder eine verminderte Abhängigkeit veränderter Zellen von diesen Wachstumsfaktoren (Rodeck et al., International Journal of Cancer 40 (1987), 687 - 690) wird es Tumorzellen ermöglicht, sich unkontrolliert zu vermehren. Die oben genannten tumorinhibierenden Faktoren aus verschiedenen Tumorgeweben stellen interessante Verbindungen dar, um in dieses gestörte Regulationssystem therapeutisch eingreifen zu können. Für eine solche therapeutische Verwendung müssen diese Faktoren in großer Menge und reproduzierbarer Reinheit zur Verfügung gestellt werden können. Bislang sind jedoch für die meisten dieser Faktoren lediglich angereicherte Fraktionen aus Zellysaten beschrieben, die auf Grund ihrer komplexen und zum Teil unbekannten Zusammensetzung und der damit verbundenen nicht reproduzierbaren Herstellbarkeit für einen therapeutischen Einsatz nicht geeignet sind.

Grundlage der Erfindung ist ein neues Melanom-inhibierendes Protein (im weiteren als MIA-Protein oder MIA bezeichnet), welches das Wachstum der Zellinien HTZ 19-dM und ATCC CRL 1424 inhibiert und

a) codiert wird von der in SEQ ID NO 1 für das reife Protein bzw. für das Protein mit N-terminaler Prä-Sequenz gezeigten DNA-Sequenz, oder der in SEQ ID NO 3 gezeigten genomischen Sequenz,

b) codiert wird von DNA-Sequenzen, die mit den in SEQ ID NO 1 oder 3 gezeigten DNA-Sequenzen oder Fragmenten dieser DNA-Sequenzen im DNA-Bereich, der für das reife Protein codiert, hybridisieren.

Unter einer Wachstuminhibierung ist eine-antiproliferative Wirkung zu verstehen. Dabei wird das Wachstum der Zellen bei Zusatz des MIA-Proteins zum Kulturmedium deutlich verlangsamt. Eine hierfür geeignete Konzentration ist beispielsweise O,1 μg MIA-Protein/ml Kulturmedium. Höhere oder geringere Konzentrationen des MIA-Proteins sind jedoch ebenfalls geeignet zur Wachstumsinhibierung, die je nach Konzentration stärker oder geringer beobachtet wird.

Die Eigenschaften eines solchen Proteine sind durch die Erfinder in Cancer Research 49 (1989), 5358 - 5363, Cancer Research 50 (1990), 6981 - 6986, Melanoma Research 2 (1992), 327 - 336 beschrieben. Ein nacharbeitbares Verfahren zur Herstellung dieses Proteins ist jedoch in diesen Publikationen nicht angegeben. Das Protein ist aus der humanen Melanomazellinie HTZ 19-dM, die bisher der Öffentlichkeit noch nicht zugänglich war, erhältlich. Diese Zellinie wurde von einem metastasierenden malignen Melanom abgeleitet und in definiertem serumfreien Kulturmedium (50 % Dulbecco's minimal essential medium, 50 % F-12) mit 0,8 mmol/l L-Glutamin, nichtessentiellen Aminosäuren, 10 μg/ml Transferrin, 30 nmol/l Natriumselenit und 4 μg/ml Gentamicin als Monolayer-Kultur unter Standard-Kulturbedingungen kultiviert. Die Zellinie wurde am 23.06.93 bei der Deutschen Sammlung für Mikroorganismen und Zellkulturen GmbH in Braunschweig hinterlegt (DSM ACC 2133). Sie ist ein weiterer Gegenstand der Erfindung. Aus dem Kulturüberstand dieser Zellinie kann das erfindungsgemäße Protein über eine gelchromatographische Isolierung einer Proteinfraktion einer Größe von ca. 11 kD und anschliessende Aufreinigung dieser Fraktion über eine reverse phase HPLC gewonnen werden.

Das Protein kann über seine DNA-Sequenz und die davon abgeleitete Aminosäuresequenz definiert werden. Das MIA-Protein kann in natürlichen allelischen Variationen, die sich von Individuum zu Individuum unterscheiden können, vorkommen (z.B. SEQ ID NO:24). Solche Variationen der Aminosäuren sind in der Regel Aminosäureaustausche. Es kann sich aber auch um Deletionen, Insertionen oder Additionen von Aminosäuren zur Gesamtsequenz handeln. Das erfindungsgemäße MIA Protein kann - sowohl in Umfang und Art abhängig von der Zelle und dem Zelltyp, in dem es exprimiert wird - glycosyliert oder nicht glycosyliert sein.

Das erfindungsgemäße Protein kann auch rekombinant hergestellt werden. Bei der rekombinanten Herstellung in Prokaryonten wird nichtglykosyliertes MIA-Protein erhalten. Mit Hilfe der durch die Erfindung bereitgestellten Nukleinsäuresequenzen ist es möglich, in Genomen von beliebigen Zellen (z. B. außer in Humanzellen auch in Zellen anderer Säugetiere) nach dem MIA-Gen oder seinen Varianten zu suchen, diese zu identifizieren und das gewünschte für das MIA-Protein codierende Gen zu isolieren. Derartige Verfahren und die hierfür geeigneten Hybridisierungsbedingungen sind dem Fachmann bekannt und beispielsweise bei J. Sambrook, E.F. Fritsch und T. Maniatis, Molecular cloning, Cold Spring Harbor Laboratory, 1989 und B.D. Hames, S.G. Higgins, Nucleic acid hybridisation - a practical approach (1985) IRL Press, Oxford, England, beschrieben. Üblicherweise werden hierbei die dort beschriebenen Standardprotokolle für die Experimente verwendet.

Durch die Anwendung der rekombinanten DNA-Technik ist es möglich, eine Vielzahl von MIA-Protein-Derivaten herzustellen. Derartige Derivate können beispielsweise modifiziert sein in einzelnen oder mehreren Aminosäuren durch Substitution, Deletion oder Addition. Die Derivatisierung kann beispielsweise über site directed mutagenesis erfolgen. Derartige Variationen sind für einen Fachmann ohne weiteres durchführbar (J. Sambrook, B.D. Hames, Loc. Lit.). Es muß lediglich sichergestellt sein, daß die charakteristischen Eigenschaften des MIA-Proteins (Inhibition der genannten Zellinien) erhalten bleiben.

Ein weiterer Gegenstand der Erfindung ist deshalb ein MIA-Protein, welches

a) das Produkt einer prokaryontischen oder eukaryontischen Expression einer exogenen DNA ist,

b) codiert wird von der in SEQ ID NO 1 für das reife Protein bzw. für das Protein mit N-terminaler Prä-Sequenz gezeigten DNA-Sequenz, der in SEQ ID NO 3 gezeigten genomischen Sequenz,

c) codiert wird von DNA-Sequenzen, die mit den in SEQ ID NO 1 oder 3 gezeigten DNA-Sequenzen oder Fragmenten dieser DNA-Sequenzen im DNA-Bereich, der für das reife Protein codiert, hybridisieren, oder

d) codiert wird von DNA-Sequenzen, die ohne die Degeneration des genetischen Codes mit den in b) bis c) definierten Sequenzen hybridisieren würden und für ein Polypeptid mit Aminosäuresequenz codieren.

Bevorzugt ist ein Protein, welches codiert wird von den Nukleotiden 40 - 432 oder 112 bis 432 aus SEQ ID NO 1 oder von DNA-Sequenzen, die aufgrund der Degeneration des genetischen Codes für ein Polypeptid mit derselben Aminosäuresequenz codieren würden.

Das MIA-Protein aus HTZ 19-dM weist ein Molekulargewicht von ca. 11 kD auf, ist stabil gegen Hitze (3 Minuten bei 100° C) und sensitiv gegenüber Proteasen, wie z. B. Trypsin.

Gegenstand der Erfindung ist eine Nukleinsäure, welche für ein MIA-Protein codiert und ausgewählt ist aus der Gruppe

a) der in SEQ ID NO 1 und 3 gezeigten DNA-Sequenzen oder den komplementären Sequenzen,

b) Nukleinsäuren-Sequenzen, die mit einer der Sequenzen von a) hybridisieren,

c) Nukleinsäuren-Sequenzen, die ohne die Degeneration des genetischen Codes mit einer der in a) oder b) genannten Sequenzen hybridisieren würden.

Ein weiterer Gegenstand der Erfindung sind melanominhibierende Proteine aus Säugerzellen, wie z.B. Maus, Ratte, Rind, Schaf, welche in im wesentlichen analoger Weise das Wachstum der Zellinien HTZ19-dM und ATCC CRL 1424 inhibieren, wie das humane MIA-Protein.

Diese dem humanen MIA-Protein analogen Proteine können dadurch erhalten werden, daß mit einer Hybridisierungsprobe, welche für humanes MIA codierende Sequenzen enthält, eine cDNA-Bibliothek des entsprechenden Säugetiers nach dem Fachmann geläufigen Methoden gescreent wird und über den Sequenzvergleich mit der DNA und Proteinsequenz für humanes und murines MIA (SEQ ID NO:1-5) das entsprechende codierende Segment identifiziert wird.

Ein bevorzugter Gegenstand der Erfindung ist das murine MIA-Protein und die hierfür codierende Nukleinsäuresequenz (SEQ ID NO:4). Das murine Protein wird codiert von den Nukleotiden 110 - 499 oder 179 - 499 von SEQ ID NO:4.

Mit Hilfe dieser Nukleinsäuren kann das erfindungsgemäße Protein in reproduzierbarer Weise in großen Mengen gewonnen werden. Zur Expression in prokaryontischen oder eukaryontischen Organismen, wie z.B. prokaryontischen Wirtszellen oder in eukaryontischen Wirtszellen, wird die Nukleinsäure nach dem Fachmann geläufigen Verfahren in geeignete Expressionsvektoren integriert. Vorzugsweise enthält ein solcher Expressionsvektor einen regulierbaren/induzierbaren Promotor. Zur Expression werden diese rekombinanten Vektoren dann nach bekannten Verfahren in geeignete Wirtszellen, wie z. B. E.coli, als prokaryontische Wirtszellen oder Saccharomyces cerevisiae, Teratocarcinoma-Zellinie PA-1 sc 9117 (Büttner et al., Mol. Cell. Biol. 11, 1991, 3573 - 3583), Insektenzellen, CHO- oder COS-Zellen als eukaryontische Wirtszellen eingeführt und die transformierten bzw. transduzierten Wirtszellen unter Bedingungen kultiviert, die eine Expression des heterologen Gens ermöglichen. Die Isolierung des Proteins kann aus der Wirtszelle oder dem Kulturüberstand der Wirtszelle nach bekannten Verfahren erfolgen. Derartige Verfahren sind beispielsweise bei Ausubel I., Frederick M., Current Protocols in Mol. Biol. (1992), John Wiley and Sons, New York, beschrieben. Es kann auch eine in vitro Reaktivierung des Proteins erforderlich sein.

Vorzugsweise wird zur rekombinanten Herstellung des erfindungsgemäßen Proteins eine DNA mit den Nukleotiden 40 - 432 oder 112 - 432 (codierende Sequenz) aus SEQ ID NO 1 (cDNA) oder die genomische DNA gemäß SEQ ID NO 3 verwendet.

Ein weiterer Gegenstand der Erfindung ist ein Verfahren zur Gewinnung eines MIA-Proteins durch Isolierung aus dem Kulturüberstand der Melanomzelline HTZ 19-dM über eine gelchromatographische Auftrennung und Aufreinigung einer Fraktion, die einem Molekulargewicht von ca. 11 kD (SDS-PAGE, nicht reduziert) entspricht, über reverse phase HPLC. Auf diese Weise können ca. 0,2 $\mu$g/l Kulturüberstand erhalten werden.

In einer bevorzugten Ausführungsform wird das natürliche MIA-Protein bei der Isolierung/Aufreinigung einer Säurebehandlung unterworfen. Dadurch kann die MIA-Aktivität gesteigert werden. Vorteilhaft ist ein pH-Wert um ca. 2, als Säure ist beispielsweise Essigsäure geeignet.

Der Nachweis von transformierten bzw. transduzierten Wirtszellen, welche das MIA-Protein rekombinant produzieren, sowie die Aufreinigung des Proteins erfolgen vorzugsweise über Antikörper, die an dieses Protein binden. Derartige Antikörper sind mit Hilfe des erfindungsgemäßen Proteins als Antigen oder Immunogen in einfacher Weise nach bekannten Verfahren erhältlich.

Ein weiterer Gegenstand der Erfindung ist daher die Verwendung des erfindungsgemäßen Proteins mit Melanominhibierender Aktivität zur Herstellung von Antikörpern, die an dieses Protein binden.

Dazu werden hierzu üblicherweise verwendete Tiere, wie insbesondere Schafe, Kaninchen oder Mäuse mit dem erfindungsgemäßen Protein immunisiert und anschließend das Antiserum der immunisierten Tiere nach bekannten Verfahren gewonnen oder Milzzellen der immunisierten Tiere mit immortalen Zellen, wie z. B. Myelomzellen, gem. dem Verfahren von Köhler und Milstein (Nature 256 (1975), 495 - 497) fusioniert. Von den so erhaltenen Hybridomzellen werden dann solche ausgewählt und kloniert, welche einen monoklonalen Antikörper gegen das MIA-Protein produzieren. Die so erhaltenen monoklonalen oder polyklonalen Antikörper können für eine immunadsorptive Reinigung des Melanom-inhibierenden Proteins an ein Trägermaterial, wie z. B. Cellulose, gebunden werden. Weiterhin können derartige Antikörper für einen Nachweis des MIA-Proteins in Proben, wie etwa Gewebeschnitten oder Körperflüssigkeiten, verwendet werden.

Ein weiterer Gegenstand der Erfindung sind daher Antikörper gegen das MIR-Protein, welche erhältlich sind durch Immunisierung eines Tieres mit einem MIR-Protein und Gewinnung der Antikörper aus dem Serum oder Milzzellen der immunisierten Tiere.

Es hat sich gezeigt, daß das MIA-Protein nicht nur auf Melanomzellen sondern auch in geringerem Umfang auf andere Tumorzellen, wie z. B. Glioblastomzellen, Neuroblastome, small cell lung cancer und neuroektodermale Tumoren eine hemmende Wirkung durch Hemmung der DNA-Synthese ($^3$H-Thymidin-Einbau (Coligan J. E., Kruisbeek A. M., Margulies D. H., Shevach E. M., Strober W., Current Protocols Immunology, NIH Monograph, J. Wiley and Sons, New York, 1992)), Hemmung einer Tumorkoloniebildung im Soft-Agar oder im Tumorstammzellen-Assay (Schlag P., Flentje D., Cancer Treatment Rev. 11: Suppl. A: 131 - 137, 1984) aufweist. Das Wachstum von normalen, nichtentarteten Zellen wird dagegen nicht inhibiert. Dabei wirkt dieses Protein bereits in sehr geringen Konzentrationen (Nanogramm-Bereich). Daher eignet sich dieses Protein für die Herstellung eines Therapeutikums zur Tumortherapie. Insbesondere ist ein solches Therapeutikum zur Therapie von malignen Melanomen, malignen Gliomen, Bronchial-Karzinomen (insbesondere kleinzelliges Bronchialkarzinom, SCLC) und Neuroblastomen geeignet.

Es hat sich weiterhin gezeigt, daß das Melanom-inhibierende Protein die Interleukin 2-abhängige bzw. Phytohämagglutinin-induzierte Proliferation peripherer Blutlymphozyten supprimiert. Weiterhin wird die Cytotoxizität von T-Lymphozyten reduziert. Das Melanom-inhibierende Protein eignet sich damit auch zur Herstellung eines Therapeutikums, das Anwendung als Immunsuppressivum findet.

Ein weiterer Gegenstand der Erfindung ist daher die Verwendung eines erfindungsgemäßen Proteins zur Herstellung eines Therapeutikums, das Anwendung in der Tumortherapie oder als Immunsuppressivum findet.

Für die genannten therapeutischen Anwendungen wird das erfindungsgemäße Protein ggf. mit den üblicherweise verwendeten Hilfs-, Füll- und/oder Zusatzstoffen zusammen in einer pharmazeutischen Formulierung verarbeitet.

Ein weiterer Gegenstand der Erfindung ist daher eine therapeutische Zusammensetzung, enthaltend ein erfindungsgemäßes Melanom-inhibierendes Protein, ggf. zusammen mit den üblicherweise verwendeten Hilfs-, Füll- und/oder Zusatzstoffen.

Ein weiterer Gegenstand der Erfindung ist die Verwendung von Sequenzen des MIA-Gens, vorzugsweise von für ein Protein mit MIA-Aktivität codierenden Sequenzen oder aktivierende Sequenzen aus dem 5' untranslatierten Bereich, in der Gentherapie und insbesondere zur Herstellung von Arzneimitteln für die Gentherapie.

Gentherapie von somatischen Zellen kann beispielsweise unter Verwendung von retroviralen Vektoren, anderen viralen Vektoren oder durch nicht-viralen Gentransfer erfolgen (zur Übersicht vgl. T. Friedmann, Science 244 (1989) 1275; Morgan 1993, RAC DATA MANAGEMENT Report, June 1993).

Gentherapeutisch geeignete Vektorsysteme sind beispielsweise Retroviren (Mulligan, R.C. (1991) in Nobel Symposium 8: Ethiology of human disease at the DNA level (Lindsten, J. and Pattersun Editors), pages 143 - 189, Raven Press), Adeno Associated Virus (McLughlin, J.-Virol. 62 (1988), 1963), Vaccinia Virus (Moss et al., Ann. Rev. Immunol. 5 (1987) 305), Bovine Papilloma Virus (Rasmussen et al., Methods Enzymol. 139 (1987) 642) oder Viren aus der Gruppe der Herpesviren, wie Epstein Barr Virus (Margolskee et al., Mol. Cell. Biol. 8 (1988) 2937) oder Herpes Simplex Virus.

Es sind auch nicht-virale Deliverysysteme bekannt. Hierzu wird üblicherweise "nackte" Nukleinsäure, vorzugsweise DNA, verwendet, oder Nukleinsäure zusammen mit einem Hilfsstoff, wie z.B. mit Transferreagenzien (Liposomen, Dendromere, Polylysin-Transferrin-Konjugate (Wagner, 1990; Felgner et al., Proc. Natl. Acad. Sci. USA 84 (1987) 7413)).

Ein weiteres bevorzugtes gentherapeutisches Verfahren basiert auf der homologen Rekombination. Dabei kann entweder das für das MIA-Protein codierende Gen in einer oder mehreren Kopien in das Genom von somatischen Zellen eingeführt werden und/oder das endogen in den Zellen vorhandene MIA-Gen moduliert, vorzugsweise aktiviert werden.

Verfahren zur homologen Rekombination sind beispielsweise beschrieben in Kucherlapati, Proc. in Nucl. Acids Res. and Mol. Biol. 36 (1989) 301; Thomas et al., Cell 44 (1986) 419-428; Thomas und Capecchi, Cell 51 (1987) 503-512; Doetschman et al., Proc. Natl. Acad. Sci. USA 85 (1988) 8583-8587 und Doetschman et al., Nature 330 (1987) 576-578. Bei diesen Verfahren wird ein Stück DNA, das im Genom an einer bestimmte Stelle integriert werden soll (Genfragment von MIA), an eine "Targeting DNA" gebunden. Die Targeting DNA ist eine DNA, die komplementär (homolog) zu einer Region der genomischen DNA ist (vorzugsweise im oder in der Nähe des MIA-Gens). Wenn zwei homologe Stücke einer Einzelstrang-DNA (z.B. die Targeting DNA und die genomische DNA) in unmittelbare Nähe zueinander kommen, hybridisieren sie und bilden eine Doppelstrang-Helix. Durch ein Rekombinationsereignis kann dann das MIA-Genfragment und die Targeting-DNA ins Genom integriert werden. Diese homologe Rekombination kann sowohl in vitro als auch in vivo (am Patienten) durchgeführt werden.

Vorzugsweise wird eine DNA verwendet, die für ein Protein mit MIA-Aktivität codiert, ein Fragment, welches die MIA-Expression inhibiert (Knock-out-Sequenz), oder ein Fragment, welches in der Lage ist, nach Integration ins Genom einer Zelle die Expression eines Proteins mit MIA-Aktivität in dieser Zelle zu aktivieren. Ein solches Fragment kann zum Beispiel eine Promotor- und/oder Enhancer-Region sein, die heterolog zur entsprechenden MIA-Region ist oder nach Integration in das MIA-Gen das an sich stumme oder gering exprimierte MIA-Gen transkriptional und/oder translatorisch aktiviert.

Mit dieser DNA werden also ein oder mehrere MIA-Gene in die Zielzelle (Targetzelle) neu eingebracht oder das im wesentlichen transkriptional stumme Gen im Genom einer Säugerzelle so aktiviert, daß die Säugerzelle befähigt wird, endogenes MIA-Protein herzustellen. Hierzu wird ein DNA-Konstrukt in das Genom durch homologe Rekombination insertiert, wobei dieses DNA-Konstrukt umfaßt: ein DNA-Regulationselement, welches in der Lage ist, die Expression dieses Gens zu modulieren, vorzugsweise zu stimulieren, wenn es operativ daran gekoppelt ist; und ein oder mehrere DNA-Target-Segmente, welche homolog zu einer Region in diesem Genom sind, die innerhalb oder in der Nähe dieses Gens liegt. Dieses Konstrukt wird in einer Weise ins Genom der Säugerzelle insertiert, daß das regulatorische Segment operativ an das Gen gekoppelt ist, welches für das Protein mit MIA-Aktivität codiert. Vorzugsweise umfaßt das Konstrukt noch amplifizierende Sequenzen, insbesondere wenn Gene, die für ein Protein mit MIA-Aktivität codieren, in die Zelle eingebracht werden.

Für die Einführung der MIA-Gene in die Zielzellen umfaßt das Konstrukt ein Regulationselement, ein oder mehrere MIA-Gene und ein oder mehrere Targetsegmente. Die Targetsegmente sind so ausgewählt, daß sie mit einer geeigneten Region des Genoms hybridisieren, wobei die insertierten exogenen MIA-Gene nach homologer Rekombination exprimiert werden.

Es sind eine Vielzahl von Verfahren bekannt, durch die homologe Rekombination initiiert werden kann. Vorzugsweise erfolgt die homologe Rekombination während der DNA-Replikation oder Mitosis der Zellen. Eine derartige DNA kann zur Herstellung eines Tumortherapeutikums oder zur Herstellung eines homologen oder heterologen MIA-Proteins in einem Wirtsorganismus verwendet werden.

Auf Basis der durch die Erfindung bereitgestellten Nukleinsäure-Sequenzen des MIA-Proteins ist es möglich, einen Test bereitzustellen, mit dem Nukleinsäuren, welche für MIA-Proteine codieren, nachgewiesen werden können. Ein derartiger Nachweis kann beispielsweise in Zellen oder Zell-Lysaten erfolgen. Ein solcher Nachweis kann mittels der Nukleinsäuren-Diagnostik geführt werden. Hierbei wird die zu untersuchende Probe (sample) mit einer Sonde in Kontakt gebracht, welche mit der für das MIA-Protein codierenden Nukleinsäuren-Sequenz hybridisieren würde. Eine Hybridisierung zwischen der Sonde und Nukleinsäuren aus der Probe zeigt das Vorhandensein von exprimierten MIA-Proteinen. Derartige Verfahren sind dem Fachmann bekannt und beispielsweise in der WO 89/06698, EP-A 0 200 362, USP 2915082, EP-A 0 063 879, EP-A 0 173 251, EP-A 0 128 018 beschrieben.

In einer bevorzugten Ausführungsform der Erfindung wird die für ein MIA-Protein codierende Nukleinsäure der Probe vor dem Test amplifiziert, beispielsweise mittels der bekannten PCR-Technik. Üblicherweise wird im Rahmen der Nukleinsäurediagnostik eine derivatisierte (markierte) Nukleinsäuresonde verwendet. Diese Sonde wird mit einer an einen Träger gebundenen denaturierten DNA oder RNA aus der Probe in Kontakt gebracht und hierbei die Temperatur, Ionenstärke, pH-Wert und sonstige Pufferbedingungen - abhängig von der Länge der Nukleinsäureprobe und der daraus resultierenden Schmelztemperatur des zu erwartenden Hybrids - so gewählt, daß die markierte DNA oder RNA an homologe DNA oder RNA binden kann (Hybridisierung, siehe auch J. Mol. Biol. 98 (1975), 503; Proc. Natl. Acad. Sci. USA 76 (1979), 3683). Als Träger geeignet sind Membranen oder Trägermaterialien auf Basis Nitrocellulose (z. B. Schleicher und Schüll, BA 85, Amersham Hybond, C.), verstärkte oder gebundene pulverförmige Nitrocellulose oder mit verschiedenen funktionellen Gruppen (z. B. Nitrogruppe) derivatisierte Nylonmembranen (z. B. Schleicher und Schüll, Nytran; NEN, Gene Screen; Amersham Hybond M.; Pall Biodyne).

Die Detektion von hybridisierender DNA oder RNA erfolgt dann dadurch, daß der Träger nach gründlichem Waschen und Absättigen zur Verhinderung unspezifischer Bindung mit einem Antikörper oder Antikörperfragment inkubiert wird. Der Antikörper oder das Antikörperfragment ist gegen die in der Nukleinsäuresonde bei der Derivatisierung inkorporierte Substanz gerichtet. Der Antikörper wiederum ist markiert. Es kann jedoch auch eine direkt markierte DNA verwendet werden. Nach der Inkubation mit den Antikörpern wird nochmals gewaschen, um nur spezifisch gebundene Antikörperkonjugate nachzuweisen. Die Bestimmung erfolgt dann über die Markierung des Antikörpers oder Antikörperfragments nach an sich bekannten Methoden.

Der Nachweis der MIA-Expression kann durchgeführt werden beispielsweise als:

- in situ-Hybridisierung mit fixierten ganzen Zellen mit fixierten Gewebesabstrichen und isolierten Metaphasen-Chromosomen,
- colony-Hybridisierung (Zellen) und plaque-Hybridisierung (Phagen und Viren),
- Northern-Hybridisierung (RNA-Nachweis),
- Serum-Analytik (z. B. Zelltypanalyse von Zellen in Serum, durch slot-blot-Analyse),
- nach Amplifikation (z.B. PCR-Technik).

Die Erfindung umfaßt daher ein Verfahren zum Nachweis von Nukıeinsäuren, welche für ein MIA-Protein codieren, dadurch gekennzeichnet, daß die zu untersuchende Probe mit einer Nukleinsäurensonde inkubiert wird, welche ausgewählt ist aus der Gruppe

a) der in Sequenz ID NO 1 und 3 gezeigten DNA-Sequenzen oder der dazu komplementären Sequenz,

b) Nukleinsäuren, die mit einer der Sequenzen von a) hybridisieren,

die Nukleinsäuresonde mit der Nukleinsäure der Probe inkubiert wird und die Hybridisierung ggf. über einen weiteren Bindepartner von Nukıeinsäure der Probe und Nukleinsäuresonde nachgewiesen wird.

MIA ist damit ein wertvoller prognostischer Marker in der Tumordiagnostik (Metastasen, Verlauf).

Die Erfindung wird durch die Sequenzprotokolle in Verbindung mit den folgenden Beispielen und Abbildungen näher erläutert. Dabei bedeuten:

SEQ ID NO 1 - cDNA von humanem MIA mit Prä-Sequenz

SEQ ID NO 2 - Protein

SEQ ID NO 3 - genomische DNA von MIA

SEQ ID NO 4 - cDNA von murinem MIA mit Prä-Sequenz

SEQ ID NO 5 - Protein

SEQ ID NO 6 - Primer

SEQ ID NO 7 - Primer

SEQ ID NO 8 - Klonierungsfragment

SEQ ID NO 9 - Primer

SEQ ID NO 10 - Primer

SEQ ID NO 11 - Adaptor

SEQ ID NO 12 - Adaptor

SEQ ID NO 13 - Fusionsprotein

SEQ ID NO 14 - Fusionsprotein

SEQ ID NO 15 - Primer

SEQ ID NO 16 - Primer

SEQ ID NO 17 - Primer

SEQ ID NO 18 - fusionsfreies MIA zur Expression in E.coli

SEQ ID NO 19 - Primer

SEQ ID NO 20 - Primer

SEQ ID NO 21 - Primer

SEQ ID NO 22 - Primer

SEQ ID NO 23 - Polylinker

SEQ ID NO 24 - genomische DNA von MIA (allelische Variante)

Fig. 1 zeigt die invasionshemmende Wirkung von humanem MIA (Hemmung der Zellwanderung mit und ohne MIA in %). B16 + mMIA: Test mit murinem MIA.

Fig. 2 Inhibition von T-Cell-vermittelter cytotoxischer Aktivität durch MIA als %Lyse von $CD4^+$ T-Zellen.

Fig. 3 Inhibition der cytotoxischen Aktivität von LAK-Zellen durch MIA.

Fig. 4 Inhibition der Phytohämagglutinin-abhängigen Lymphozytenproliferation durch MIA (MIA-Konzentration in ng/ml).

Fig. 5 Inhibition der IL-2-stimulierten PBMC Proliferation durch MIA (MIA-Konzentration in ng/ml).

Fig. 6 Plasmidkarte des Expressionsplasmids pQE40-MIA (Beispiel 5a).

Fig. 7 Plasmidkarte des Vektors pCMX-PL1 (Beispiel 7a).

**Beispiel 1**

**Isolierung des Melanom-inhibierenden Proteins aus HTZ 19-dM-Zellen.**

HTZ 19-dM-Zellen werden als Monolayer in definiertem serumfreien Gewebekulturmedium (50 % Dulbecco's minimal essential medium, 50 % F-12, Boehringer Mannheim GmbH) mit 0,8 mmol/l L-Glutamin (Gibco, U.K.), nichtessentiellen Aminosäuren (Gibco, U.K.), 10 µg/ml Transferrin (Boehringer Mannheim GmbH, Katalog-Nr. 1073974), 30 nmol/l Natriumselenit (Sigma) und 4 µg/ml Gentamycin (Merck) kultiviert. Der Zellkulturüberstand dieser Kultur wird im Abstand von jeweils 3 - 4 Tagen abgenommen und bis zur Aufreinigung bei -70°C gelagert.

Zur Aufreinigung werden die Zellkulturüberstände durch 0,45 µm Filter (Becton Dickinson, Heidelberg) filtiert und durch Membran-Ultrafiltration mit Amicon YM 2-Membranen (Auschlußgrenze 2000 D, Amico Danvers Massachusetts, USA) auf ein Endvolumen von 1 % des Ausgangsvolumens aufkonzentriert. Das erhaltene Material wird für 30 Stunden gegen 0,1 mol/l Essigsäure dialysiert (Dialysemembran mit Ausschlußgrenze von 1000 D, Reichelt, Heidelberg) und anschließend bei 100 000 g für eine Stunde bei 4°C ultrazentrifugiert. Das Pellet wird verworfen und der Überstand zur weiteren Verarbeitung lyophilisiert.

Die lyophilisierten Dialysate werden in 1 mol/l Essigsäure aufgenommen und durch Gelpermeationschromatographie an einer Biogel P-10-Säule (Pharmacia, Uppsala, 2,6 x 100 cm; Biogel $P^{-10}$, 200 - 400 Mesh, Biorad Laboratories, Richmond, California, USA) weiter aufgereinigt. Das Gelmaterial wird mit 1 mol/l Essigsäure bei 22°C äquilibriert und die Dialysate in einer Konzentration von 130 - 145 mg in 5 ml in 1 mol/l Essigsäure aufgetragen. Eluiert wird mit 1 mol/l Essigsäure bei einer Flußrate von 12 ml/Std. und das Eluat in 4 ml-Fraktionen aufgefangen. Über Bestimmung der Anti-Tumor-Aktivität (vgl. Beispiel 5) werden drei aktive Fraktionspools definiert, von denen der mittlere Pool, der einem Molekulargewicht von 8000 - 17000 D entspricht, über reverse phase HPLC weiter aufgereinigt wird. Dazu werden diese Fraktionen zunächst einmal lyophilisiert und dann in 0,1 % Trifluoressigsäure (TFA) aufgenommen. Jeweils 100 µl Aliquots der erhaltenen Lösung werden auf die reverse phase HPLC aufgetragen (Flußrate 0,5 ml/min). Es werden Fraktionen mit je 750 µl gesammelt. Weitere Daten der HPLC-Trennung:

Gradientenprogramm: Lösung A: 0,06 % TFA in Wasser
Lösung B: 0,056 % TFA, 80 % Acetonitril

2 - 25 % Lösung B in 5 min
25 - 50 % Lösung B in 120 min
50 - 100 % Lösung B in 5 min
zurück auf 2 % in 5 min
Säule: minoRPC (Pharmacia)
HPLC-Gradientenmischer, Pumpe und Detektor: Pharmacia

Das Eluat wird in 1,5 ml-Fraktionen aufgefangen. Aliquots werden lyophilisiert und, wie in Beispiel 5 beschrieben, auf Anti-Tumor-Wirkung hin untersucht.

Auf diese Weise können ca. 1 µg Melanom-inhibierendes Protein aus 5 l Kulturüberstand gewonnen werden.

**Beispiel 2**

**Beispiel 2a**

**Klonierung der für das humane Melanom-inhibierende Protein codierenden cDNA**

Nach Asp-N und Trypsin-Verdau des gereinigten Proteins und erneuter Aufreinigung der so erhaltenen Peptidfragmente werden die MIA-Aminosäuresequenzen im Sequenzierer bestimmt. Die C-terminale und eine nahe dem N-Terminus gelegene Peptidsequenz wurden als Grundlage zur Synthese zweier Primer ausgewählt. Bei den Primern handelt es sich um degenerierte Oligonukleotide mit angefügten Restriktionsenzymschnittstellen.

Upstream Primer 1 (sense) (UP 1) (SEQ ID NO:6)

5'TGTGAATTCAGTTIA/TG/CIGCIGAT/CCAA/GGAA/GTG 3'
EcoRI site

Der Querstrich (/) bedeutet, daß entweder die Base vor oder hinter dem Querstrich an dieser Stelle steht. Dieses Oligonukleotid ist eine Mischung aus 32 verschiedenen Molekülen, wodurch fast alle möglichen Codons abgedeckt sind. Lediglich an den Positionen 12 und 13 sind G-T Mismatches mit der Zielsequenz möglich, was die Stabilität der Hybride nicht erhöht, aber auch nicht vermindert. Am 5'-Ende hängt zusätzlich ein EcoRI-Linker, um ein eventuelles Produkt nach PCR leicht umklonieren zu können. Davor liegen am 5'-Ende noch 3 unspezifische Basen, damit die Restriktionsschnittstelle nicht ganz am Rand liegt, da Restriktionsenzyme dort weniger gut schneiden.

Downstream Primer 1 (Antisense) (DP1) (SEQ ID NO:7)

5'TGTGTCGACTGTTCGTAGAAA/GTCCCATCTTA/GTC 3'
SalI site

DP 1 entspricht den 8 C-terminalen Aminosäuren, ist 8-fach degeneriert und enthält eine Sal I Schnittstelle.
Für die spezifische Erststrangsynthese wurde der Primer DP 1 in dem folgenden Ansatz verwendet:

| | |
|---|---|
| 5 µl | 10 x PCR Puffer |
| 3 µl | HTZ-19 Gesamt-RNA |

wurden gemischt und 5 min auf 65°C erhitzt. Dazu wurden pipettiert:

| | |
|---|---|
| 1 µl | 100 mM $MgCl_2$ |
| 10 µl | 2,5 mM dNTP |
| 0,5 µl | Placenta RNase Inhibitor |
| 2 µl | DP 1 (1 µg) |
| 1 µl | Reverse Transkriptase |

Nach 1 h Inkubation bei 37°C wurde zu obigem Ansatz folgendes zugegeben:

| | |
|---|---|
| 1 µl | UP 1 (1 µg) |
| 5 µl | 10 x PCR Puffer |
| 70,5 µl | $H_2O$ |
| 1 µl | Taq-Polymerase |

Die Amplifikation erfolgte 30 Zyklen mit dem folgenden Profil:

| | |
|---|---|
| 94°C | 30 sec. |
| 55°C | 30 sec. |
| 72°C | 60 sec. |

Nach dem letzten Zyklus wurde der Ansatz zur vollständigen Verlängerung aller Produkte noch 7 min. bei 72°C inkubiert.
Nach einer Phenol/Chloroform-Extraktion und EtOH-Fällung erfolgte der Verdau des PCR-Ansatzes mit EcoRI und SalI 2 h bei 37°C. Nach Enzymaktivierung folgte die Auftrennung in einem 5 % PAA-Gel und Elution des 320 bp großen Fragmentes über Nacht. Die Hälfte der Eluats wurde über Nacht mit 100 ng EcoRI/SalI verdautem pbluescript ligiert. Von den am nächsten Tag transformierten und auf SOB-Agar-Platten mit Amp und X-Gal/IPTG ausplattierten Bakterien (E. coli DH5a) konnte eine rekombinante weiße Kolonie gepickt werden.
Nach Isolation des Plasmids erfolgte die Sequenzierung des umklonierten Inserts mit dem T-7 Deaza Sequencing-Kit von Pharmacia. Als Primer standen der T-3 und T-7 Primer (Stratagene) zur Verfügung. Aus der Überlappung der abgelesenen Sequenzen ergab sich folgendes Bild (Primer fett gedruckt):

(SEQ ID NO: 8)

```
GAATTC AAG TTT TCG GCG GAT CAG GAG TGC AGC CAC CCT ATC TCC ATG
EcoRl  Lys Phe Ser Ala Asp Gln Glu Cys Ser His Pro Ile Ser Met

GCT GTG GCC CTT CAG GAC TAC ATG GCC CCC GAC TGC CGA TTC CTG ACC
Ala Val Ala Leu Gln Asp Tyr Met Ala Pro Asp Cys Arg Phe Leu Thr

ATT CAC CGG GGC CAA GTG GTG TAT GTC TTC TCC AAG CTG AAG GGC CGT
Ile His Arg Gly Gln Val Val Tyr Val Phe Ser Lys Leu Lys Gly Arg

GGG CGG CTC TTC TGG GGA GGC AGC GTT CAG GGA GAT TAC TAT GGA GAT
Gly Arg Leu Phe Trp Gly Gly Ser Val Gln Gly Asp Tyr Tyr Gly Asp

CTG GTC GCT CGC CTG GGC TAT TTC CCC AGT AGC ATT GTC CGA GAG GAC
Leu Val Ala Arg Leu Gly Tyr Phe Pro Ser Ser Ile Val Arg Glu Asp

CAG ACC CTG AAA CCT GGC AAA GTC GAT GTG AAG ACA GAT AAA TGG GAT
Gln Thr Leu Lys Pro Gly Lys Val Asp Val Lys Thr Asp Lys Trp Asp

TTC TAC GAA CAGTCGAC
Phe Tyr Glu  SalI
```

Für die Klonierung der vollständigen cDNA stand eine Lambda gt11 cDNA-Bibliothek zur Verfügung, die aus der RNA von in definiertem Medium (dM) wachsenden HTZ-19 Melanomzellen synthetisiert worden war.

Insgesamt wurden 25 Platten mit je 8000 pfu ausplattiert, je 2 Nitrocellulose-Filter aufgelegt und mit dem durch Nick-Translation markierten gereinigten MIA-PCR Insert hybridisiert (50 ml Hybridisierungslösung, 2 x $10^6$ cpm/ml). Nach zweitägiger Autoradiographie konnten mehrere Signale erhalten werden, die auf beiden Filtern ein übereinstimmendes Hybridisierungssignal ergaben. Die zugehörigen Phagen-Plaques wurden isoliert und einer Nachuntersuchung unterzogen. Dabei wurden von jedem isolierten Plaque 4 Verdünnungsstufen ausplattiert und die Platten mit 100-300 pfu für zwei weitere Hybridisierungen verwendet.

Aus den so isolierten Plaques konnte nach einer 50 ml Übernacht-Kultur die Lambda-DNA isoliert werden, 40 µg mit EcoRI verdaut und in einem 5% PAA-Gel aufgetrennt werden. Das Insert wurde eluiert und 8 ng für die Ligation mit 100 ng EcoRI verdautem, dephosphoryliertem Vektor (pbluescript, Stratagene) eingesetzt. Die Hälfte der Ligationsansätze wurden für die Transformation kompetenter E. coli DH5a eingesetzt, die auf SOB/Amp-Platten mit IPTG und X-Gal für die Blau/Weiß Selektion ausplattiert wurden. Die rekombinanten Kolonien wurden abgeimpft, die Plasmid-DNA isoliert und die Inserts sequenziert. Die Sequenz des Inserts mit der vollständigen codierenden Sequenz ist unter SEQ ID NO 1 gezeigt.

Ein auf diese Weise erhaltenes Plasmid ist pbs L7MIA, welches bei der Deutschen Sammlung von Mikroorganismen und Zellkulturen GmbH (DSM) in Braunschweig, Deutschland am 14.07.93 hinterlegt wurde (DSM 8420).

Alle für die Klonierung verwendeten Methoden sind ausführlich in J. Sambrook, E.F. Fritsch, T. Maniatis (1989), Molecular cloning: a laboratory manual, 2nd edition, Cold Spring Harbor Laboratory Press, Cold Spring Harbor, USA, beschrieben.

**Beispiel 2b**

**Klonierung des für das humane Melanom-inhibierende Protein codierenden Gens**

Eine humane genomische DNA Bibliothek im Bacteriophagen Lamoda FIX II (Elgin et al. (1991), Strategies 4, 8-9), kommerziell erhältlich von Stratagene (Heidelberg), wurde nach etablierten Methoden (Sambrook et al. Molecular Cloning, Cold Spring Harbor Laboratory, 1989) auf Nitrocellulose-Filter plattiert. Zur Verwendung als Hybridisierungs-Probe wurde die für humanes MIA codierende cDNA aus Beispiel 2a radioaktiv markiert und entsprechend etablierten Techniken verwendet (Sambrook et al. (1989), Molecular Cloning, Cold Spring Harbor Laboratory Press). Vorhybridisierung (2 Stunden) und Hybridisierung (16 Stunden) erfolgten bei 60°C in 6 x SSC, 5 x Denhardt's Lösung, 100 µg ml Lachssperma-DNA und 0.1% SDS. Die $^{32}$P-dCTP-markierte Probe wurde dem Hybridisierungsansatz in einer Konzentration von 1 x $10^6$ cpm/ml zugesetzt. Anschließend wurden die Filter bei 60°C zweimal 20 Minuten gewaschen in 2 x SSC, 0.1 % SDS, dann zweimal 20 Minuten in 1 x SSC, 0.1 % SDS und schließlich zweimal 20 Minuten in 0.25 x SSC, 0.1 % SDS. Die Filter wurden daraufhin getrocknet und 24 bis 48 Stunden auf Roentgenfilm exponiert. Die bei diesem Verfahren ein positives Hybridisierungssignal liefernden Plaques wurden isoliert und durch Rehybridisierung bestätigt. Die in diesen Phagen als Insert enthaltene humane genomische DNA wurde durch Southern-Hybridisierung unter Verwendung von MIA cDNA-Proben charakterisiert. Hierzu wurde die Phagen DNA mit der Restriktionsendonuklease XbaI gespalten, auf einem 0.8 % Agarose-Gel aufgetrennt und anschließend nach Southern (J.Mol.Biol. 98 (1975), 503) auf Nitrocellulose transferiert. Die so erhaltenen Filter wurden unter den oben beschriebenen Bedingungen mit der kompletten MIA cDNA als Probe hybridisiert, wobei zwei XbaI Fragmente von ca. 1.4 kb und ca. 2.2 kb positive Signale lieferten. Diese beiden Fragmente wurden jeweils in das zur Sequenzierung geeignete und bei Stratagene (Heidelberg) käuflich erhältliche Plasmid pBluescriptSK- (Short et al. (1988), Nucl. Acids res. 16, 7583 - 7600; Alting-Meese und Short (1989), Nucl. Acids res. 17, 9494) kloniert. Die gesamte, für das humane MIA codierende Sequenz ist in vier Exons lokalisiert, die in SEQ ID NO:3 mit ihren flankierenden Sequenzen gezeigt sind. Da nicht untersucht wurde, ob zwischen den beiden XbaI-Fragmenten, auf denen die MIA Exons lokalisiert sind, sich eines oder sogar mehrere weitere XbaI-Fragmente befinden, kann nicht ausgeschlossen werden, daß Intron 2 in Wirklichkeit wesentlich größer ist.

**Beispiel 2c**

**Klonierung der für das murine Melanom-inhibierende Protein codierenden cDNA**

Eine kommerziell erhältliche (Novagene, New York) cDNA-Bibliothek aus einem 13.5 Tage alten Maus-Embryo im Vektor Lamoda EXlox (Palazzolo et al. (1990), Gene 88, 25 - 36) wurde wie in Beispiel 2a beschrieben plattiert. Als Hybridisierungsprobe wurde die für humanes MIA codierende cDNA aus Beispiel 2a in radioaktiv markierter Form eingesetzt. Die Hybridisierungsbedingungen waren identisch mit den in Beispiel 2b beschriebenen, mit Ausnahme der Temperaturen bei Hybridisierung und Waschung, die hier 55°C betrugen. Die in den so erhaltenen und durch Rehybridisierung bestätigten Plaques vorliegenden cDNA-Inserts wurden sequenziert. Die Sequenz des Inserts mit der vollständigen codierenden DNA des murinen MIA ist in SEQ ID NO 4 gezeigt.

**Beispiel 2d**

**Klonierung des für das murine Melanom-inhibierende Protein codierenden Gens**

In diesem Fall wurde eine murine genomische DNA-Bibliothek (aus der Leber einer adulten BALB/C Maus im Vektor EMBL3 (Frischauf et al. (1983), J.Mol.Biol. 170, 827), käuflich erhältlich bei Clontech, Palo Alto CA) in analoger Weise wie in Beispiel 2b beschrieben unter Verwendung der murinen MIA cDNA aus Beispiel 2c als Probe abgesucht. Die Bedingungen waren identisch zu den in Beispiel 2b beschriebenen. Auch das weitere Vorgehen erfolgte in analoger Weise.

**Beispiel 3**

**Beispiel 3 a**

**Bestimmung der antiproliferativen Wirkung des Melanominhibierenden Proteins auf Tumorzellen**

Zur Bestimmung der antiproliferativen Wirkung des Melanom-inhibierenden Proteins bzw. der gemäß Beispiel 1 erhaltenen Proteinfraktionen auf Melanomzellen werden exponentiell wachsende HTZ 19-dM-Zellen für 24 Stunden in 96er Mikrokulturplatten (Costar, Zürich) in jeweils 100 µl serumfreiem Medium (s. Beispiel 1) mit einer Dichte von 3 x $10^3$-Zellen pro Vertiefung ausgesät (gemäß Chambard et al., J. Cell. Physiol. 135 (1988), 101 - 107). Die Zellen werden dann mit der zu untersuchenden Proteinfraktion für 4 - 5 Tage bei 37°C/5 % $CO_2$ inkubiert. Nach Zugabe von jeweils 1 µCi $^3$H Thymidin (spezifische Aktivität 23 Ci/mmol, Amersham Buchler, Braunschweig, Deutschland) werden die Zellen 8 Stunden unter identischen Bedingungen weiter inkubiert und anschließend der $^3$H Thymidin-Einbau in die zelluläre DNA nach Säurefällung in üblicher Weise über einen Flüssigszintillationszähler gemessen. Die Aktivität der untersuchten Proteinfraktion wird als Prozentsatz des $^3H$ Thymidin-Einbaus dieser behandelten Zellen, gegenüber dem $^3H$ Thymidin-Einbau in unbehandelten Kontrollzellen ausgedrückt. Unter Verwendung verschiedener Konzentrationen des isolierten Melanom-inhibierenden Proteins kann eine Konzentration ermittelt werden, bei welcher dieser $^3H$ Thymidin-Einbau um 50 %, gegenüber der unbehandelten Kontrolle, gehemmt wird (IC 50-Wert in der folgenden Tabelle 1).

Tabelle 1

| Antiproliferative Wirkung des Melanom-inhibierenden Proteins auf verschiedene Tumorzellen | |
|---|---|
| Tumorzellinie | IC 50 (µg/ml)[1)] |
| a) Melanomzellinien | |
| HTZ 19-dM | 1,2 |
| ATCC HTB 69 | 3,7 |
| HTZ 320 | 1,35 |
| HTZ 318 | 3,5 |
| ATCC CRL 1424 | 2,1 |
| b) Neuroblastomlinien | |
| Kelly | 80 |
| c) Glioblastom | |
| ATCC HTB17 | 10 |
| d) Astrocytom | |
| HTZ 243 | 5 |
| HTZ 209 | 5 |

[1)] Verwendet wurde das Protein nach dem ersten Reinigungsschritt (nach BiogelP10 Säule, Faktor 50-100 geringere Aktivität als nach vollständiger Reinigung)

**Beispiel 3b**

**Bestimmung der invasionshemmenden Wirkung des Melanominhibierenden Proteins auf Tumorzellen**

Zur Bestimmung der invasionshemmenden Wirkung von MIA wird ein modifiziertes Boyden-Kammer-System (Albini et al. (1987), Cancer Res. 47, 3239 - 3245) verwendet. Die Kammern wurden von der Firma Costar (Blind Well Chamber No. 441200) bezogen. Zur Simulierung einer Basalmembran-ähnlichen Barriere zwischen dem Chemoattraktans in der unteren Kammer und den Zellen in der oberen Kammer werden auf den Polycarbonatfilter (Porengröße 8 µm, Costar No. 150446) 52 µl Matrigel (Becton Dickinson Cat. No. 40234) aufgebracht. Als Chemoattraktans wird die untere Kammer mit 210 µl Fibroblasten-konditioniertem Medium befüllt. Dieses wird wie folgt gewonnen: Fibroblasten aus normaler menschlicher Haut werden zwischen der 10. und 20. Passage für 24 Stunden in DMEM-Medium (Gibco) ohne Zusatz von foetalem Kälberserum gehalten. Das so konditionierte Medium wird unverdünnt als Chemoattraktans eingesetzt. In die obere Kammer der Boyden-Apparatur werden 2 x $10^5$ der zu untersuchenden Tumorzellen in 800 µl DMEM (Gibco, ohne foetales Kälberserum) mit, bzw. ohne MIA-Wirkstoff gegeben. Humane Tumorzellen (siehe Beispiel 3a oder Beispiel 8) oder tierische Tumorzellen wie etwa B16 (ATCC CRL 6322) können mit der beschriebenen Methode auf Inhibierung ihres Migrationsverhaltens durch MIA getestest werden. Ohne Zugabe des Melanom inhibierenden Proteins wandern ca 10 % Tumorzellen innerhalb von ca 4 Stunden aus der oberen Kammer in die untere Kammer, wo sie an der Unterseite der Matrigel-Membran haften bleiben. Sie werden dort fixiert, gefärbt und anschließend gezählt. Wird der oberen Kammer das humane oder murine Melanom inhibierende Protein MIA zugesetzt, so wird die Wanderung der Zellen stark inhibiert. Fig. 1 zeigt die erhaltenen Hemmwerte ([Zellzahl in der unteren Kammer, Experiment mit MIA] / [Zellzahl in der unteren Kammer, Experiment ohne MIA] x 100 % ).

**Beispiel 4**

**Bestimmung der immunologischen Aktivität**

**Beispiel 4a**

**MIA hemmt T-Zell vermittelte zytotoxische Aktivität**

Die gegen ein Peptid aus myelin basic protein (MBP, Aminosäureposition 87 - 106) spezifische $CD4^+$ T-Zellinie D7.1 ist in der Lage, in einem Standard $^{51}Cr$-Release Assay sowohl MBP- als auch Peptid 87-106 präsentierende Targets zu lysieren (Targets: Daudi-Zellen, R. Martin, U. Utz, J.E. Coligan, J.R. Richert, M. Flerlage, E. Robinson, R. Stone, W.E. Biddison, D.E. McFarlin, H.F. MacFarland, Diversity in fine specificity and T cell receptor usage of the human $CD^{4+}$ cytotoxic T-cell response specific for the immunodominant myelin basic protein peptide 87 - 106, J. Immunol. (1992), 148 (5), 1359 - 1366). Nach Zugabe von MIA (eingesetzte Menge entspricht ca. 50 - 100 ng/ml gereinigten MIA) wird die Peptid-spezifische Zytotoxizität um ca. 55 % (Fig. 2 a), die MBP-spezifische Zytotoxizität um ca. 50 % (Fig. 2 b) gehemmt. Die Hemmung zeigt erwartungsgemäß eine geringe Abhängigkeit vom Effektor-Target-Verhältnis (E:T), das hier mit 1:1 bzw. 5:1 sehr niedrig angesetzt wurde und damit hochspezifisch ist (Fig. 2).

**Beispiel 4b**

**MIA hemmt die zytotoxische Aktivität von Lymphokinaktivierten peripheren Blutlymphozyten (LAK-Zellen)**

LAK-Zellen sind nicht klonal expandierte, Lymphokin-aktivierte periphere Blutlymphozyten, vorwiegend T-Lymphozyten (A.A. Rayner, E.A. Grimm, M.T. Lotze, E.W. Chu, S.A. Rosenberg, Lymphokine activated killer (LAK) cells: Analysis of factors relevant for immunotherapy of human cancer, Cancer 55 (1985), 1327 - 1333). Sie werden in vielen immunologischen Therapie-Ansätzen der Tumortherapie standardisiert eingesetzt. In dem hier gezeigten Experiment werden LAK-Zellen auf ihre Zytotoxizität gegenüber HTZ-19 Melanom-Zellen als Targets in einem Microzytotoxizitäts-Assay geprüft. In Effektor-Target-Verhältnissen von 1:1, 5:1 und 10:1 kommt es zu einer maximalen Zytotoxizität (CTX) von knapp 40 %. Diese wird nach Zugabe von MIA (Konzentration wie in Beispiel 4 a) stark gehemmt, maximal um 80 % bei niedrigem Effektor-Target-Verhältnis, wie es am ehesten lokal - am Tumor selbst - zu erwarten ist (Fig. 3).

**Beispiel 4c**

**MIA hemmt die IL-2 abhängige bzw. Phytohämagglutininabhängige Lymphozytenproliferation**

Periphere Blutlymphozyten (PBMC) lassen sich klassischerweise mit Phythämagglutinin (PHA) und Interleukin-2 (IL-2) standardisiert stimulieren (J.E. Coligan, A.M. Kruisbeek, D.H. Margulies, E.M. Shevach, W. Strober, Current protocols in immunology, NIH Monograph, J. Wiley Sons, New York, 1992). Hierbei werden mit PHA fast ausschließlich T-Zellen stimuliert, mit IL-2 vorwiegend T-Lymphozyten, aber auch B-Zellen mit IL-2-Rezeptor. Unter Co-Inkubation mit MIA läßt sich im angegebenen Dosierbereich (Proteineinheit: nach dem ersten Reinigungsschritt (Biogel P10-Säule), Aktivität ist um den Faktor 50 - 100 geringer als nach vollständiger Reinigung) eine sehr starke Hemmung der PHA-Antwort erreichen (Fig. 4). Die IL-2-Antwort wird im höheren Dosisbereicht gehemmt (Fig. 5).

**Beispiel 5**

**Rekombinante Expression von MIA als Fusionsprotein in E.coli**

**Beispiel 5a**

**Konstruktion von Expressionsvektoren**

Zur Expression von MIA als Fusionsprotein mit einem als "Carrier" in E.coli geeigneten Protein kann beispielsweise der käuflich erhältliche Vektor pQE40 (Cat. No. 33403, DIAGEN GmbH, Düsseldorf) verwendet werden. In diesen Vektor wird zwischen die je einmal vorhandenen Restriktionsschnittstellen SphI und HindIII ein für die reife Form von MIA codierendes cDNA-Fragment insertiert. Ein derartiges Fragment wird am einfachsten durch PCR-Amplifikation nach bekannten Techniken hergestellt unter Verwendung der klonierten MIA cDNA als Matrize und zweier geeigneter Primer (5'-GATGCATGCGGTCCTATGCCCAAGCTG-3' (SEQ ID NO:9) und 5'-GATAAGCTTTCACTGGCAGTAGAAATC-3' (SEQ ID NO:10)). Das erhaltene PCR-Fragment wird mit SphI und HindIII geschnitten und in den ebenso behandelten Vektor pQE40 ligiert. Das resultierende Plasmid exprimiert ein Fusionsprotein aus DHFR (Dihydrofolat-Reduktase als "Carrier") und MIA. Um aus diesem Fusionsprotein freies MIA proteolytisch abspalten zu können, wird ein DNA-Segment zwischen DHFR und MIA einkloniert, welches für die Erkennungssequenz der IgA-Protease (Ser Arg Pro Pro/Ser) codiert. Dies geschieht durch Öffnen des Expressionsplasmides mit BglII (Partialrestriktion mit anschließender Isolierung des linearisierten Vektors) und SphI und anschließende Insertion eines Adaptors (5'-GATCTAGCCGGCCGCCCAGCCCGGCATG-3' (SEQ ID NO:11) und 5'-CCGGGCTGGGCGGCCGGCTA-3' (SEQ ID NO:12) hybridisiert zum Doppelstrang). Das resultierende Expressionsplasmid pQE40-MIA codiert für ein Fusionsprotein aus DHFR und MIA, wobei sich zwischen DHFR und MIA eine Spaltstelle für IgA-Protease befindet. Das Fusionsprotein trägt am N-Terminus 6 Histidine, die den Angaben des Herstellers zufolge zur Aufreinigung des Fusionsproteins mit Hilfe von Ni-Chelat-Gelmaterialien verwendet werden können. Derartige Verfahren sind in EP-A 0 282 042 und EP-A 0 253 303, deren Inhalt Gegenstand der Offenbarung dieser Anmeldung ist, beschrieben. Fig. 6 zeigt das Expressionsplasmid pQE40-MIA.

Der Gehalt von MIA läßt sich optimieren, indem das Carrier-Protein DHFR eliminiert und durch ein möglichst kleines Peptid ersetzt wird, welches dieselben Funktionen erfüllt. Derartige geeignete Peptide sind beispielsweise MetArgGlySerHisHisHisHisHisHisGlySerSerArgProPro (SEQ ID NO:13) (dieses Peptid kann durch IgA-Protease von der unmittelbar darauf folgenden Aminosäure-Sequenz des reifen MIA abgespalten werden; derartige Verfahren sind in der WO 91/11520 beschrieben, deren Inhalt Gegenstand der Offenbarung dieser Anmeldung ist) oder MetArgGlySerHisHisHisHisHisHisGlySerValAspAspAspAspLys- (SEQ ID NO:14) (dieses Peptid kann durch Enterokinase von der unmittelbar darauf folgenden Aminosäure-Sequenz des reifen MIA abgespalten werden). Expressionsplasmide, die für derartige MIA-Peptidfusionen codieren, können wie folgt hergestellt werden: Eine PCR-Amplifikation mit MIA cDNA (Seq.ID No 1) als Matrize und den Primern 5'-AAAAAGGATCCAGCCGGCCGCCCGGTCCTATGCCCAAGCTGGC-3' (SEQ ID NO:15) und 5'-GGCGAGCAGCCAGATCTCCATAG-3' (SEQ ID NO:16) liefert ein Fragment, welches mit BamHI und BglII nachgeschnitten wird. Der Expressionsvektor pQE40-MIA wird ebenfalls mit BamHI und BglII restringiert, das kleinere der entstehenden Fragmente wird verworfen und durch das beschriebene PCR-Fragment ersetzt. Hierdurch wird ein Expressionsvektor erhalten, der nach Induktion das Fusionsprotein MetArgGlySerHisHisHisHisHisHisGlySerSerArgProPro-MIA (SEQ ID NO:13) exprimiert. Das Fusionsprotein MetArgGlySerHisHisHisHisHisHisGlySerValAspAspAspAspLys-MIA (SEQ ID NO:14) wird in völlig analoger Weise unter Verwendung der Primer 5'-AAAAAAGGATCCGTTGATGATGACGATAAAGGTCCTATGCCCAAGCTGGC-3' (SEQ ID NO:17) und 5'-GGCGAGCAGCCAGATCTCCATAG-3' (SEQ ID NO:16) erhalten.

Weitere, ähnliche Fusionsproteine aus Peptiden und MIA können in analoger Weise hergestellt und unter der Kontrolle geeigneter (vorzugsweise starker und induzierbarer) Promotoren in eines der zahlreichen für E.coli beschriebenen Plasmide einkloniert und zur Expression gebracht werden. Eine weitere Alternative stellt die Fusion von MIA mit einem Peptid dar, welches in E.coli zu Sekretion des Fusionsproteines ins Periplasma mit nachfolgender Abspaltung und Freisetzung von MIA führt. Dieses Verfahren ist in WO 88/09373 beschrieben, deren Inhalt Gegenstand der Offenbarung dieser Anmeldung ist.

**Beispiel 5b**

**Expression von Fusionsproteinen und Gewinnung von MIA**

Das Expressionsplasmid pQE40-MIA (oder ein ähnliches Expressionsplasmid, welches beispielsweise durch Verwendung eines anderen Grundvektors oder eines anderen "Carrier"-Proteins oder -Peptides erhalten werden kann; derartige Alternativen sind außer in Beispiel 5a auch beschrieben in Methods of Enzymology 185 (Gene Expression Technology), Hrsg. David V. Goeddel, Academic Press 1991) wird in einen geeigneten E.coli Stamm transfiziert, der über eine hinreichende Expression des lac-Repressors verfügt, so daß eine induzierbare Expression des MIA-Fusionsproteins erzielt werden kann. Geeignet hierfür ist beispielsweise der Stamm E.coli M15[pREP4], der zusammen mit pQE40 käuflich erhältlich ist (Diagen GmbH, Düsseldorf), oder aber andere E.coli-Stämme wie etwa E.coli UT5600 (Earhart et al. (1979), FEMS Microbiology Letters 6, 277-280) oder E.coli BL21 (Grodberg und Dunn (1988), J.Bacteriol. 170, 1245-1253), die zuvor mit einem lac-Repressor exprimierenden Helfer-Plasmid wie etwa pUBS520 (Brinckmann et al. (1989), Gene 85, 109-114) oder pUBS500 (EP-B 0 373 365) transfiziert wurden. MIA kann dann wie folgt gewonnen werden: E.coli M15 [pREP4/pQE40-MIA] wird auf LB-Medium bis zu einer optischen Dichte von 0.6 (gemessen bei 550 nm) kultiviert, dann IPTG (Isopropyl-β-D-thiogalactopyranosid, Boehringer Mannheim GmbH) in einer Endkonzentration von 1 mM zugegeben und anschließend 4 Stunden weiterkultiviert. Die Zellen werden abzentrifugiert, in 100 mM Natrium-Phosphat-Puffer pH 7.5 mit 300 mM NaCl aufgenommen und durch dreimaliges Einfrieren und Auftauen mit anschließender Ultraschallbehandlung lysiert. Zu dem durch Zentrifugation geklärten Lysat wird Ni-NTA-Agarose (Diagen GmbH) unter Berücksichtigung der vom Hersteller angegebenen maximalen Bindekapazität zugegeben und über Nacht bei Raumtemperatur unter Mischen inkubiert. Das so mit Fusionsprotein beladene Gelmaterial wird niedertourig abzentrifugiert, zweimal mit 100 mM Natrium-Phosphat-Puffer pH 7.5 und zweimal mit Natrium-Phosphat-Puffer pH 6.1 gewaschen. Anschließend wird MIA aus dem Fusionsprotein abgespalten durch Inkubation des Gelmateriales mit IgA-Protease (Boehringer Mannheim GmbH) in 100 mM Natrium-Phosphat-Puffer pH 7.5 über Nacht bei 37°C. Das Gelmaterial wird durch Zentrifugation abgetrennt und der MIA-haltige Überstand nach Sterilfiltration in den in den Beispielen 3 und 4 beschriebenen Aktivitätstests eingesetzt.

**Beispiel 6**

**Rekombinante Expression von fusionsfreiem MIA in E.coli**

Die für MIA codierende DNA-Sequenz wird in einer Weise modifiziert, die eine effiziente Expression in E.coli ermöglicht. Zu diesem Zweck wird eine PCR-Amplifikation mit humaner MIA cDNA (Seq. ID No 1) als Matrize und den Primern 1 (5'-AAAAACATATGGGACCAATGCCAAAATTAGCAGATCGTAAATTATGTGCAGATCAGGAG-3' (SEQ ID NO:19) und 2 (5'-AAAAAAAGCTTTCACTGGCAGTAGAAATC-3' (SEQ ID NO:20)) durchgeführt. Primer 1 verändert die MIA codierende Sequenz in dem N-terminalen Bereich so, daß bei unveränderter MIA-Aminosäure-Sequenz die DNA- und damit die mRNA-Sequenz für E.coli optimiert wird. Das Startcodon Met wird hinzugefügt und an eben dieser Stelle eine Erkennungssequenz der Restriktionsendonuklease NdeI eingebracht, die eine anschließende Klonierung des so modifizierten MIA codierenden Fragmentes in einen Vektor gestattet, der einen (vorzugsweise starken und induzierbaren) Promoter und eine Translations-Inititations-Sequenz ("Shine-Dalgarno-Sequenz") mit nachfolgend plazierter NdeI-Schnittstelle enthält. Primer 2 dient als 3'-Gegenprimer und enthält eine HindIII-Schnittstelle, so daß die Insertion des modifizierten MIA codierenden Fragmentes in den Vektor als NdeI HindIII Fragment erfolgen kann. Die derart modifizierte MIA codierende Sequenz ist in SEQ ID NO:18 gezeigt. Ein derart hergestelltes Expressionsplasmid ist p11379, welches unter der Hinterlegungsnummer DSM 9267 am 29.06.94 bei der Deutschen Sammlung von Mikroorganismen und Zellkulturen GmbH, D-38124 Braunschweig, hinterlegt wurde.

Ein Expressionsplasmid für fusionsfreies MIA wird zur Expression in einen geeigneten E.coli Stamm transfiziert. Bei Verwendung eines durch lac-Repressor kontrollierten Expressionsplasmides wie p11379 sind dies Stämme, die über eine hinreichend hohe intrazelluläre Konzentration an lac-Repressor verfügen. Solche Stämme können durch Transfektion eines zweiten Plasmides wie pREP4 (Diagen GmbH), pUBS500 oder pUBS520 (Brinckmann et al. (1989), Gene 85, 109-114) hergestellt werden. Die verwendeten E.coli-Stämme sollten vorzugsweise eine geringe zelleigene Protease-Aktivität aufweisen, wie dies beispielsweise bei E.coli UT5600 (Earhart et al. (1979), FEMS Microbiology Letters 6:277-280), bei E.coli BL21 (Grodberg und Dunn (1988), J.Bacteriol. 170:1245-1253) oder bei E.coli B der Fall ist. Die Expressionskultivierung erfolgt dann analog wie in Beispiel 5b beschrieben. Zur Gewinnung von MIA wird das als Proteinaggregat aus E. coli erhaltene MIA nach den in EP 241 022, EP 364 926, EP 219 874 und DE 40 37 196 beschriebenen Verfahren aufgearbeitet.

Im Detail wird hierzu beispielsweise wie folgt vorgegangen: MIA-haltige Proteinaggregate aus E.coli-Fermentationen (sogenannte "Inclusion Bodies") werden in 6 M Guanidinium-Hydrochlorid, 100 mM TrisHCl pH 8, 1 mM EDTA solubilisiert, anschließend auf pH 3-4 eingestellt und gegen 4 M Guanidinium-Hydrochlorid pH 3,5 dialysiert. Die Renaturierung des solubilisierten Proteins erfolgt dann in 1 M Arginin pH 8, 1 mM EDTA, 5 mM GSH (Glutathion reduziert) und 0,5 mM GSSG (Glutathion oxidiert). Aus dem Renaturierungsansatz kann MIA beispielsweise nach Zusatz von 1,4 M Ammoniumsulfat durch Adsorption an hydrophobe Gelmatrices wie Fractogel TSK Butyl (E. Merck, Darmstadt) und anschließende Elution in 20 mM TrisHCl pH 7 gewonnen werden.

**Beispiel 7**

**Rekombinante Expression von MIA in eukaryontischen Zellen**

**Beispiel 7a**

**Rekombinante Expression von MIA in Säugerzellen**

Hierzu werden die humane (SEQ ID NO:1) oder murine (SEQ ID NO:4) MIA cDNA oder die entsprechenden genomischen DNA-Segmente in einen Vektor ligiert, in welchem sie ausgehend von einem starken Promoter-Enhancer-System in Säugerzellen transkribiert werden (im Falle der genomischen MIA Fragmente ist dieser Schritt notwendig, da die MIA eigenen Promotoren nur in bestimmten Zelltypen, beispielsweise Melanomen, aktiv und daher nicht für eine allgemeine rekombinante Expression geeignet sind. Eine Expression kann jedoch auch wie in Beispiel 9 beschrieben durch homologe Rekombination in vitro erreicht werden. Derartige Promotoren und Enhancer stammen zumeist aus Viren wie SV40, hCMV, Polyoma oder Retroviren. Alternativ können auch Promoter-Enhancer-Systeme verwendet werden, die für einen bestimmten Zell- oder Gewebetyp spezifisch sind, wie etwa WAP-, MMTV- oder Immunglobulin-Promoter, oder aber solche Systeme, die induzierbar sind, wie etwa Metallothionein-Promoter. Ein solcher Vektor ergänzt die MIA-cDNA (falls eine solche verwendet wird) mit Donor- und Akzeptor-Signalen für die RNA-Prozessierung sowie einem Signal für poly-A-Addition. Ein solcher geeigneter Vektor ist beispielsweise pCMX-pL1 (Umesono et al. (1991), Cell 65, 1255-1266), der in Fig. 7 dargestellt ist. In die einzige EcoRI-Schnittstelle dieses Vektors wird die mit EcoRI-Linkern versehene MIA cDNA ligiert, wobei durch Restriktionsanalyse mit Hilfe der übrigen Schnittstellen im Polylinker dieses Vektors (siehe SEQ ID NO:23) sichergestellt wird, daß die Orientierung der MIA-cDNA in Ableserichtung des CMV-Promoters vorliegt. Völlig analog wird bei einer Klonierung in andere Vektoren vorgegangen, z.B. in pCDNA3 (Invitrogen, San Diego/USA) oder pSG5 (Stratagene, LaJolla/USA). Die DNA der so erhaltenen Expressionsplasmide wird aus E.coli präpariert und mit für den jeweiligen Zelltyp spezifischen Techniken in die Säugerzellen transfiziert (Methods of Enzymology 185 (Gene Expression Technology), Hrsg. David V. Goeddel, Academic Press 1991, Sektion V). Das Expressionsplasmid pCMX-pL1-MIA wird in die humane Teratocarcinoma-Linie PA-1sc9177 (Büttner et al. (1991), Mol. Cell. Biol. 11, 3573-3583) nach beschriebenen Methoden (Büttner et al. (1993), Mol. Cell. Biol. 13, 4174 - 4185) transfiziert, wobei 200 000 Zellen pro 100 mm Kulturschale mit 5 µg DNA transfiziert werden. Die Zellen werden nach der Transfektion in MEM (Gibco) ohne Zusatz von foetalem Kälberserum kultiviert, wobei nach 48 Stunden im Zellkulturüberstand MIA nachweisbar ist.

**Beispiel 7b**

**Rekombinante Expression von MIA in Insektenzellen**

Zur Expression in Insektenzellen wird ein für MIA codierendes DNA-Segment, vorzugsweise die humane MIA cDNA (SEQ ID NO 1), in Vektoren eingebracht, die sich von AcMNPV (Autographa californica nuclear polyhedrosis virus) oder BmNPV (Bombyx mori nuclear polyhedrosis virus) ableiten. Hierzu wird die MIA cDNA zunächst unter die Kontrolle eines für Insektenzellen geeigneten, starken Promoters gebracht (O'Reilly DR, Miller LK und Luckow VA 1992; Baculovirus Expression Vectors - A Laboratory Manual; W.H.Freeman & Co, New York), wie des polH-Promoters oder des p10-Promoters. Zur Expression von MIA mit Hilfe des polH-Promoters wird wie folgt vorgegangen:

Ein für MIA codierendes DNA-Fragment mit Schnittstellen für die Restriktionsendonukleasen EcoRI (benachbart dem 5'-Ende des späteren MIA-Transkriptes) und PstI (benachbart dem 3'-Ende des späteren MIA-Transkriptes) wird durch PCR-Amplifikation nach bekannten Techniken unter Verwendung der MIA cDNA als Matrize und der Primer 5'-CGTGAATTCAACATGGCCCGGTCCCTGGTGTGC-3' (SEQ ID NO:21) und 5'-TATCTGCAGTCACTGGCAGTAGAAATCCCA-3' (SEQ ID NO:22) erhalten. Dieses Fragment wird mit EcoRI und PstI nachgeschnitten (zur Erzeugung der entsprechenden kohäsiven Enden) und in den mit denselben Endonukleasen restringierten Transfer-Vektor pVL1393 (O'Reilly DR, Miller LK und Luckow VA 1992; Baculovirus Expression Vectors - A Laboratory Manual; W.H.Freeman & Co, New York), der kommerziell erhältlich ist (PharMingen, San Diego, CA oder Invitrogen Corporation, San Diego, CA), einligiert. Das resultierende Transfer-Expressionsplasmid pVL1393-MIA wird zur Vermehrung in E.coli K12 transfiziert und Plasmid-DNA nach etablierten Methoden präpariert. Der Transfer der unter Kontrolle des polH-Promoters stehenden MIA-DNA vom Transfer-Plasmid in den Baculovirus-Vektor erfolgt durch homologe Rekombination nach etablierten Methoden (O'Reilly et al. 1992, siehe oben). Hierzu werden 0.5 µg BaculoGold DNA (linearisierte AcNPV Virus-DNA mit Lethal-Deletion und durch den polH-Promoter gesteuerter lacZ Expression, käuflich erhältlich bei PharMingen, Bestellnummer 21100D) und 2 µg pVL1393-MIA gemischt, 5 Minuten bei Raumtemperatur inkubiert und anschließend mit 1 ml 125 mM Hepes pH 7.1, 125 mM CaCl2 , 140 mM NaCl versetzt. Diese Mischung wird zu 2 x $10^6$ SF9-Insektenzellen (Invitrogen, Bestellnummer B825-01) in einer Kulturschale von 60 mm Durchmesser gegeben, die zuvor mit 1 ml Grace's Medium mit 10 % foetalem Kälberserum überschichtet worden waren. Nach 4 Stunden Inkubation bei 4°C wird das DNA-haltige Medium entfernt und die Zellen werden in frischem Medium für 4 Tage bei 27°C inkubiert. Die auf diesem Weg erhaltenen rekombinanten Baculoviren werden anschließend zweimal über Plaque-Bildung gereinigt (O'Reilly et al. 1992, siehe oben), wobei Viren, die MIA durch homologe Rekombination insertiert haben, sich durch das Fehlen von β-Galactosidase-Aktivität (optisch erkennbar durch das Fehlen einer Blaufärbung in Gegenwart von 5-Brom-4-chlor-3-indolyl-β-D-galactopyranosid) von den verwendeten Wildtyp-Viren (AcNPV mit Lethal-Deletion und durch den polH-Promoter gesteuerter lacZ Expression, käuflich erhältlich bei PharMingen, Bestellnummer 21100D) unterscheiden. Mit einem derart erhaltenen, MIA-exprimierenden rekombinanten Baculovirus werden nach etablierten Methoden (O'Reilly et al. 1992, siehe oben) SF9-Zellen infiziert (MOI = 20 pfu/Zelle) und mindestens 36 Stunden bei 27°C in serumfreiem Medium (Cell/Perfect Bac serum free insect cell culture medium, Stratagene, Bestellnummer 205120) weiterinkubiert. Anschließend werden die Zellkulturüberstände abgenommen, im Überstand befindliche Viren durch Ultrazentrifugation entfernt (Beckmann Ti 60 Rotor, 30000 rpm) und der Überstand anschließend durch einen Microcon 100 Filter (Amicon, Ausschlußgrenze 100 kD) filtriert. Die so erhaltene MIA-haltige Lösung kann in den in Beispiel 3 und 4 beschriebenen Tests eingesetzt oder gemäß Beispiel 1 weiter aufgereinigt werden.

**Beispiel 8**

**Nachweis von MIA mRNA in verschiedenen Zellen**

Ein Nachweis, daß in einer bestimmten Zelle MIA exprimiert wird und demzufolge MIA mRNA vorhanden ist, kann einerseits mit den etablierten Methoden der Nucleinsäure-Hybridisierung erfolgen, wie etwa Northern-Hybridisierung, in-situ-Hybridisierung, Dot- oder Slot-Hybridisierung und daraus abgeleiteten diagnostischen Techniken (Sambrook et al. 1989, Molecular Cloning - A Laboratory Manual; Cold Spring Harbor Laboratory Press; Nucleic Acid Hybridisation - A Practical Approach, Hrsg. BD Hames und SJ Higgins (1985), IRL Press; WO 89/06698, EP-A 0 200 362, USP 2915082, EP-A 0 063 879, EP-A 0 173 251, EP-A 0 128 018). Andererseits können Methoden aus dem vielfältigen Repertoire der Amplifikationstechniken unter Verwendung von MIA-spezifischen Primern angewandt werden (PCR Protocols - A Guide to Methods and Applications; Hrsg. MA Innis, DH Gelfand, JJ Sninsky, TJ White (1990), Academic Press Inc; PCR - A Practical Approach; Hrsg. MJ McPherson, P Quirke, GR Taylor (1991), IRL Press). Die Tabellen 2A und 2B zeigen die MIA Expression in verschiedenen humanen Tumoren, Tumorlinien und normalen Zellen, die in diesem Fall durch Northern-Hybridisierung unter Verwendung der radioaktiv markiertem humanen MIA cDNA (Seq. ID No. 1) ermittelt wurde. Die RNA wurde hierfür aus den aufgelisteten Zellen nach der Methode von Chomczynski und Sacchi (Anal. Biochem. 162 (1987), 156-159) isoliert. 20 µg Gesamt-RNA wurden auf einem 1% Agarose-Formaldehyd-Gel aufgetrennt und auf Nylon-Membranen (Amersham, Braunschweig) nach Standard-Methoden übertragen (Sambrook et al. (1989); Molecular Cloning - A Laboratory Manual; Cold Spring Harbor Laboratory Press). Als Probe wurde die komplette humane MIA cDNA (Seq. ID No. 1) radioaktiv markiert (Feinberg und Vogelstein (1984), Anal. Biochem. 137, 266-267). Die Hybridisierung erfolgte bei 68°C in 5 x SSC, 5 x Denhardt, 0.5 % SDS, 10 % Dextransulfat und 100 µg/ml Lachssperma-DNA. Anschließend wurden die Membranen zweimal je eine Stunde in 1 x SSC bei 68°C gewaschen und dann auf Roentgenfilm exponiert.

Tabelle 2A

| Tumor | Anzahl getestet | positiv im Northern Blot |
|---|---|---|
| Astrocytom | 10 | 3 |
| Oligodendrogliom | 4 | 0 |
| Ependymom | 3 | 0 |
| Neuroblastom | 4 | 0 |
| Glioblastom | 13 | 0 |
| Colon-CA | 2 | 1 |
| malignes Melanom ZNS-Metastase | 6 | 6 |
| Medulloblastom | 2 | 0 |
| Mamma-CA | 1 | 0 |
| Bronchial-CA ZNS-Metastase | 2 | 0 |

Tabelle 2B

| Normalzellen | Anzahl getestet | positiv im Northern Blot |
|---|---|---|
| embryonale Fibroblasten | 2 | 0 |
| mononukleäre Blutzellen (3 Spender) | 3 | 0 |

**Beispiel 9**

**Verwendung von MIA codierenden Nukleinsäuren zu therapeutischen Zwecken**

MIA hemmt die Proliferation und die Metastasierung von Tumorzellen. Dieser Effekt kann in einem Tiermodell oder einem Patienten nicht nur durch die exogene Zufuhr von MIA-Protein hervorgerufen werden, sondern auch durch das Einbringen eines DNA-Segmentes, das entweder für MIA unter einem geeigneten Promoter codiert oder aber einen geeigneten Promoter enthält, der durch homologe Rekombination vor das zelleigene MIA Gen in das Genom integrieren kann. In letzterem Fall muß dieser Promoter von Sequenzabschnitten flankiert sein, die den Sequenzen des humanen (oder im Falle eines Tiermodells tierischen) MIA Gens im 5'-untranslatierten Bereich möglichst weitgehend homolog oder vorzugsweise identisch sind (WO 91/09955).

Mit diesem Verfahren läßt sich erreichen, daß die Tumorzelle vermehrt MIA exprimiert und so die eigene Proliferation und ihre Metastasierung inhibiert, sofern die DNA-Segmente in die Tumorzelle selbst eingebracht werden. In vielen Fällen wird es jedoch nicht erforderlich sein, die entsprechenden Gensegmente spezifisch und ausschließlich in die Tumorzellen selbst einzubringen, da auch eine Expression in anderen, vorzugsweise dem Tumor benachbarten, Körperzellen über eine vermehrte MIA-Ausschüttung eine Inhibition der Tumorzellen bewirkt. Im folgenden Beispiel ist die therapeutische Wirkung eines MIA-codierenden DNA-Segmentes in einem Tiermodell gezeigt.

Die Injektion von murinen B16-Melanom-Zellen (ATCC CRL 6322) in die Schwanzvene von C57BL-Mäusen mit nachfolgender Quantifizierung von Lungenmetastasen stellt ein etabliertes in vivo Metastasierungsmodell dar. 100.000 Zellen der Melanomlinie B16 wurden retrobulbär in C57BL-Mäuse injiziert (Tag 1, 16 Tiere) Nach 48 Stunden wurden bei 8 Tieren je 100 $\mu$g des MIA-Expressionsplasmides pCMX-PL1-MIA (Beispiel 7a) in TE (10 mM TrisCl pH 8.0, 1 mM EDTA) gemischt mit DOTAP Transfektionsreagenz (Leventis und Silvius (1990), Biochim.Biophys.Acta 1023, 124-132, kommerziell erhältlich bei Boehringer Mannheim, Cat.No 1202375), in die Schwanzvene injiziert. Die Kontrollgruppe (8 Tiere) erhielt dasselbe Plasmid, jedoch ohne MIA-Sequenzen. Nach 13 Tagen hatten in der Kontrollgruppe ohne MIA 6 von 8 Tieren einen lokalen Tumor entwickelt; die durchschnittliche Zahl der Metastasen in Lunge, Milz, Niere und Leber betrug 7.8. In der Gruppe, die das MIA codierende Plasmid erhalten hatte, entwickelten nur 4 von 8 Tieren einen lokalen Tumor und die durchschnittliche Zahl der Metastasen betrug 2.7.

## Patentansprüche

1. Verwendung von expressionsaktivierenden Nukleinsäurefragmenten aus dem 5'-untranslatierten Bereich von SEQ ID NO:3 zur Herstellung von viralen oder nicht-viralen Vektoren für die Gentherapie.

2. Verwendung nach Anspruch 1, dadurch gekennzeichnet, daß das Nukleinsäurefragment eine Promotor- und/oder Enhancer-Region ist.

3. Verwendung nach Anspruch 1, dadurch gekennzeichnet, daß das Fragment nach Integration ins Genom einer eukaryontischen Zelle die Expression eines Proteins mit MIA-Aktivität aktivieren kann.

Fig. 1

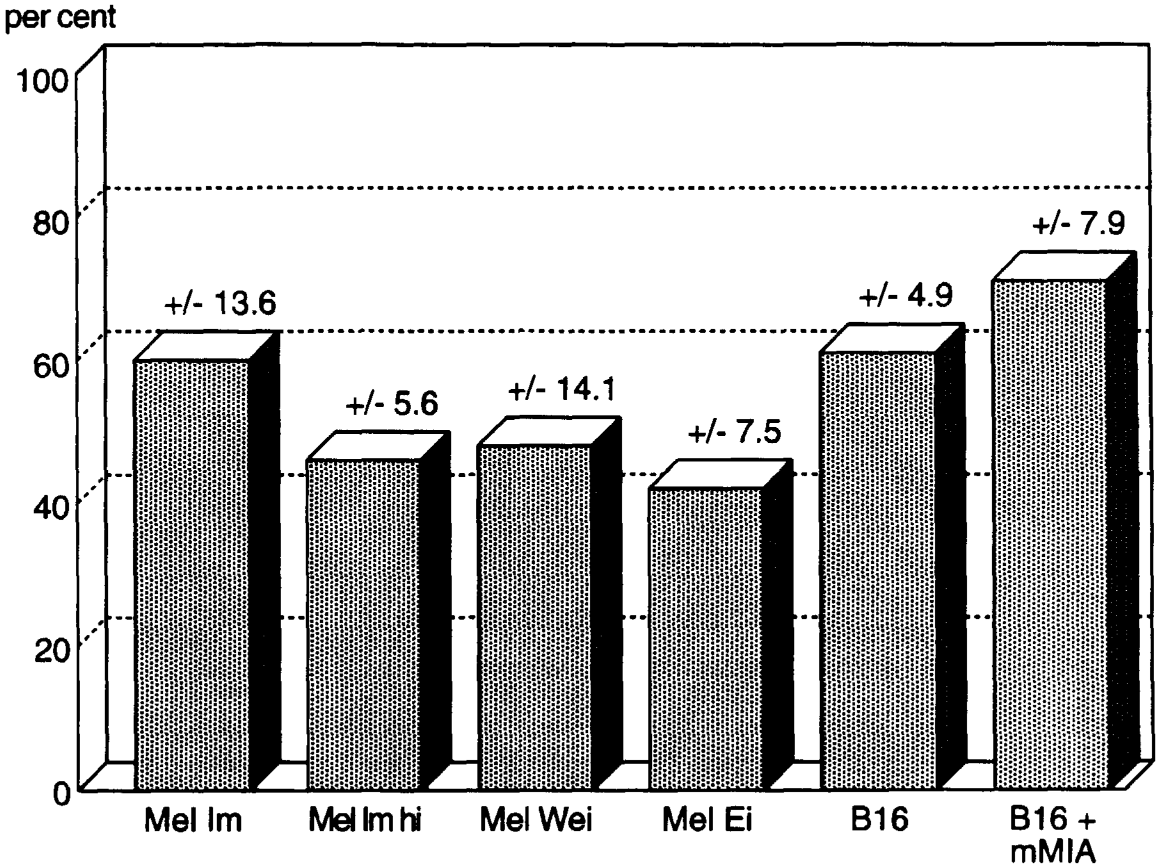
per cent
100
80
60
40
20
0
+/- 13.6
+/- 5.6
+/- 14.1
+/- 7.5
+/- 4.9
+/- 7.9
Mel Im
Mel Im hi
Mel Wei
Mel Ei
B16
B16 + mMIA

Fig. 2

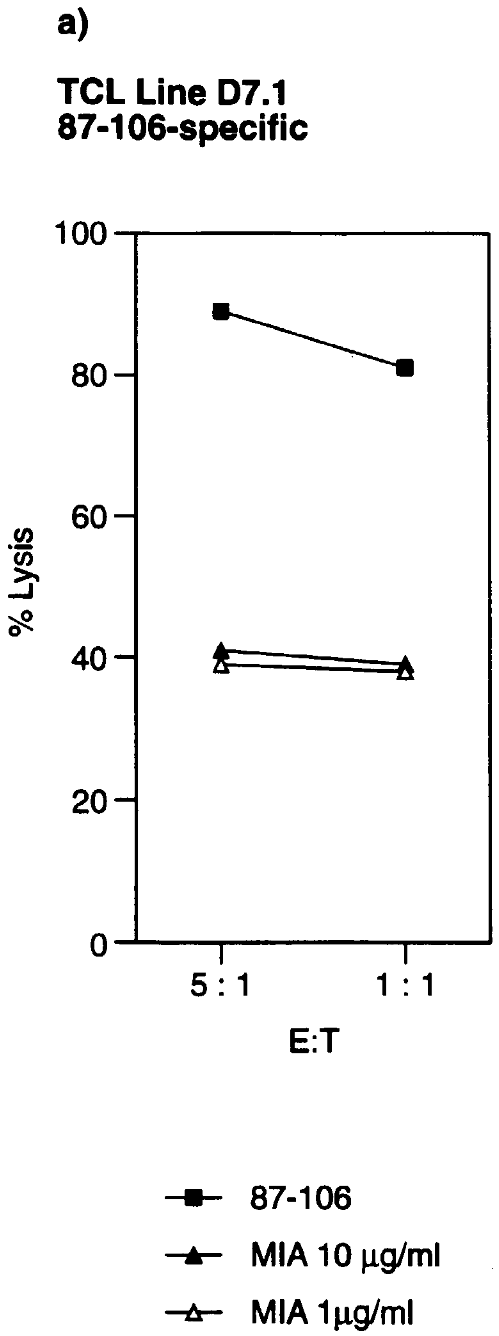
a)
TCL Line D7.1
87-106-specific
100
80
60
40
20
0
% Lysis
5 : 1
1 : 1
E:T
87-106
MIA 10 µg/ml
MIA 1µg/ml

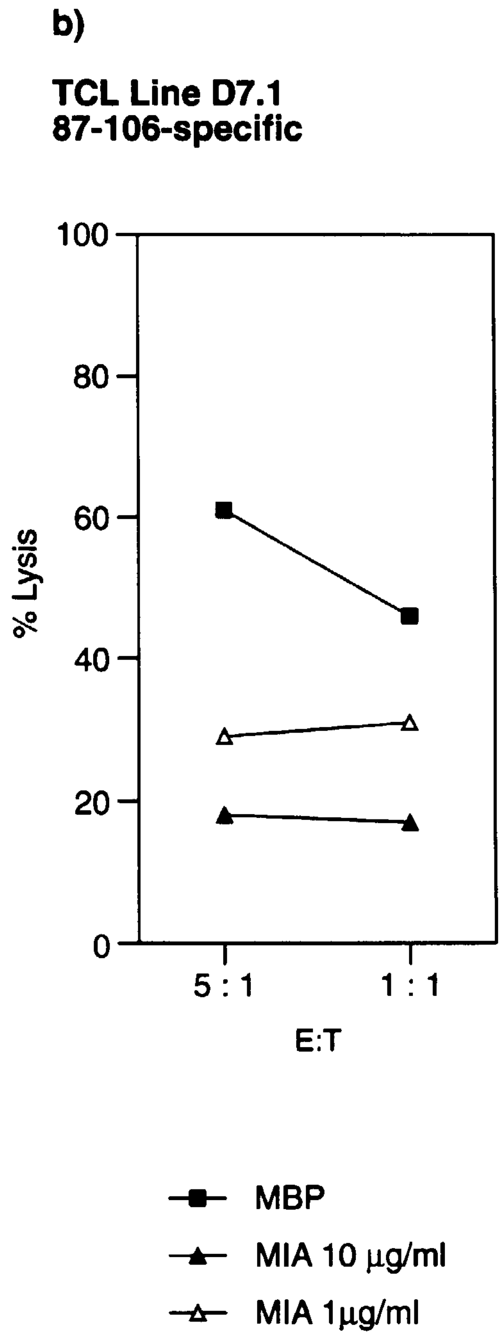
b)
TCL Line D7.1
87-106-specific
100
80
60
40
20
0
% Lysis
5 : 1
1 : 1
E:T
MBP
MIA 10 µg/ml
MIA 1µg/ml

Fig. 3

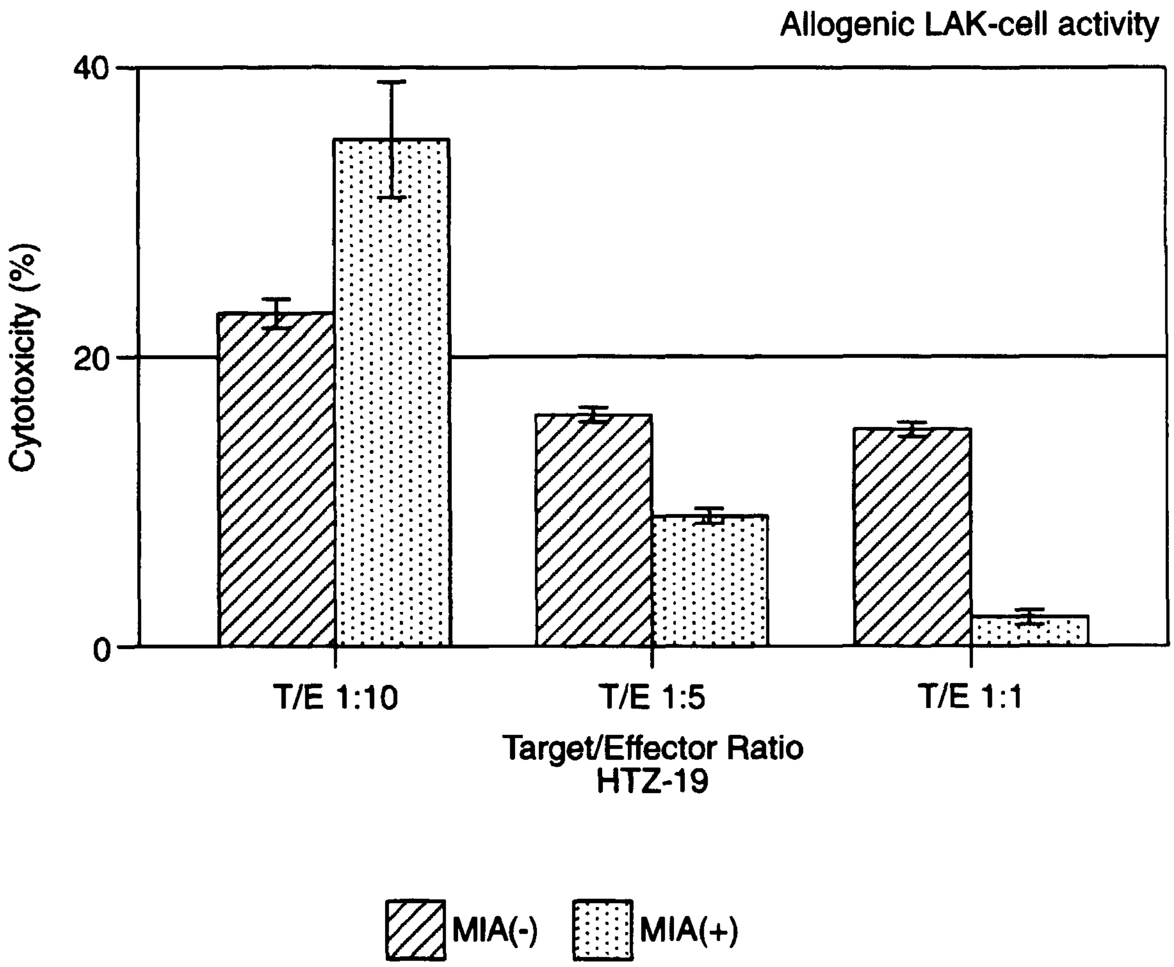
Allogenic LAK-cell activity
40
20
0
Cytotoxicity (%)
T/E 1:10
T/E 1:5
T/E 1:1
Target/Effector Ratio
HTZ-19
MIA(-)
MIA(+)

**Fig. 4**

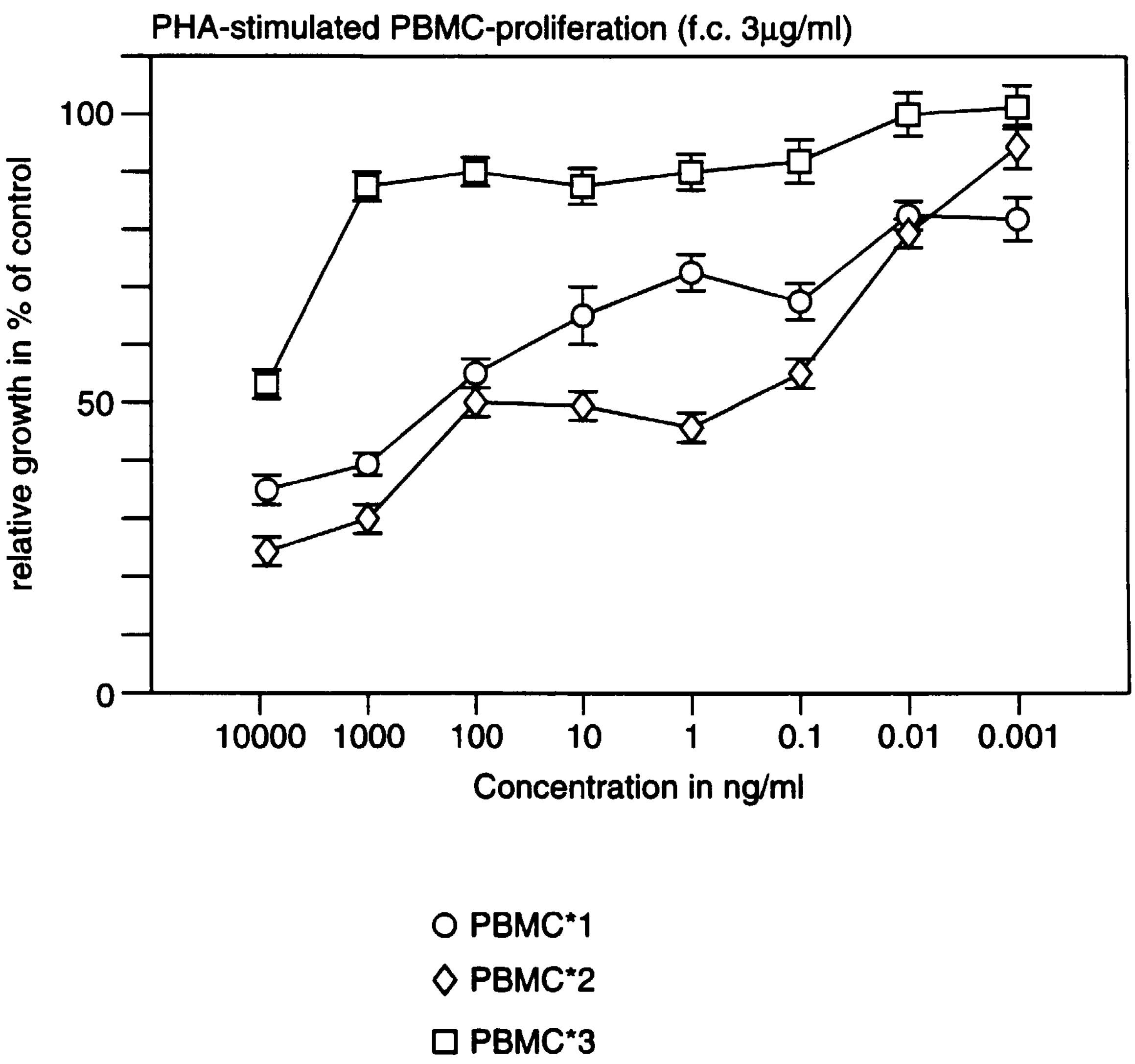
PHA-stimulated PBMC-proliferation (f.c. 3µg/ml)
relative growth in % of control
100
50
0
10000
1000
100
10
1
0.1
0.01
0.001
Concentration in ng/ml
PBMC*1
PBMC*2
PBMC*3

Fig. 5

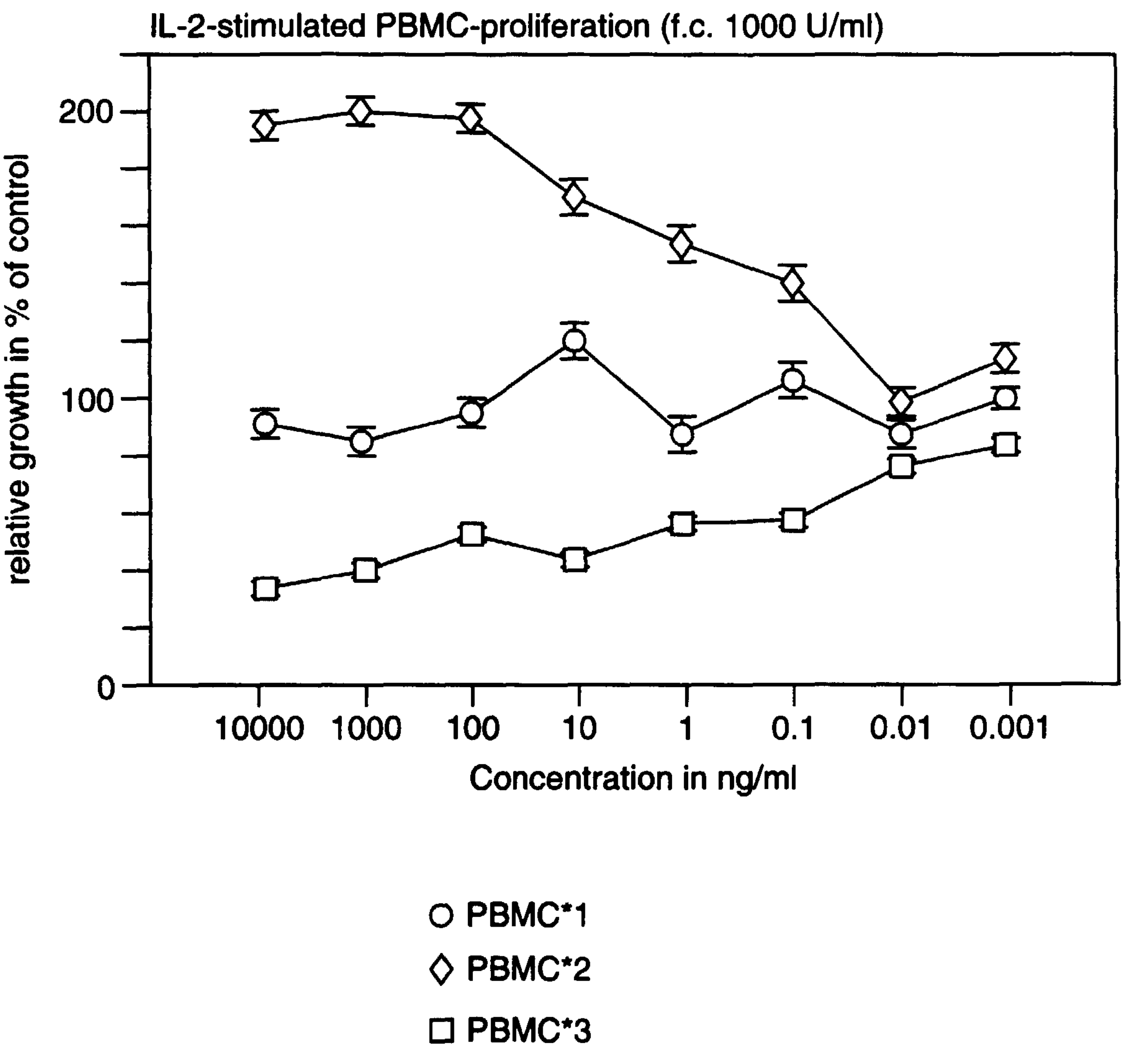
IL-2-stimulated PBMC-proliferation (f.c. 1000 U/ml)
relative growth in % of control
200
100
0
10000
1000
100
10
1
0.1
0.01
0.001
Concentration in ng/ml
PBMC*1
PBMC*2
PBMC*3

Fig. 6

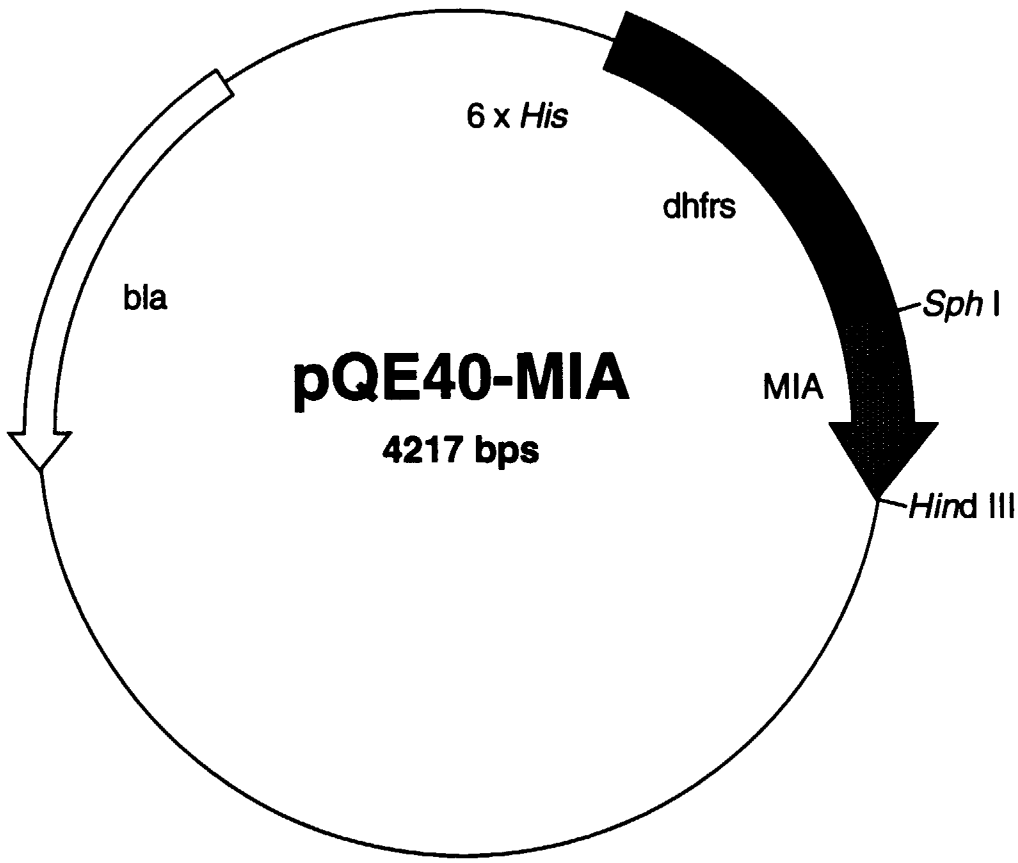

Fig. 7

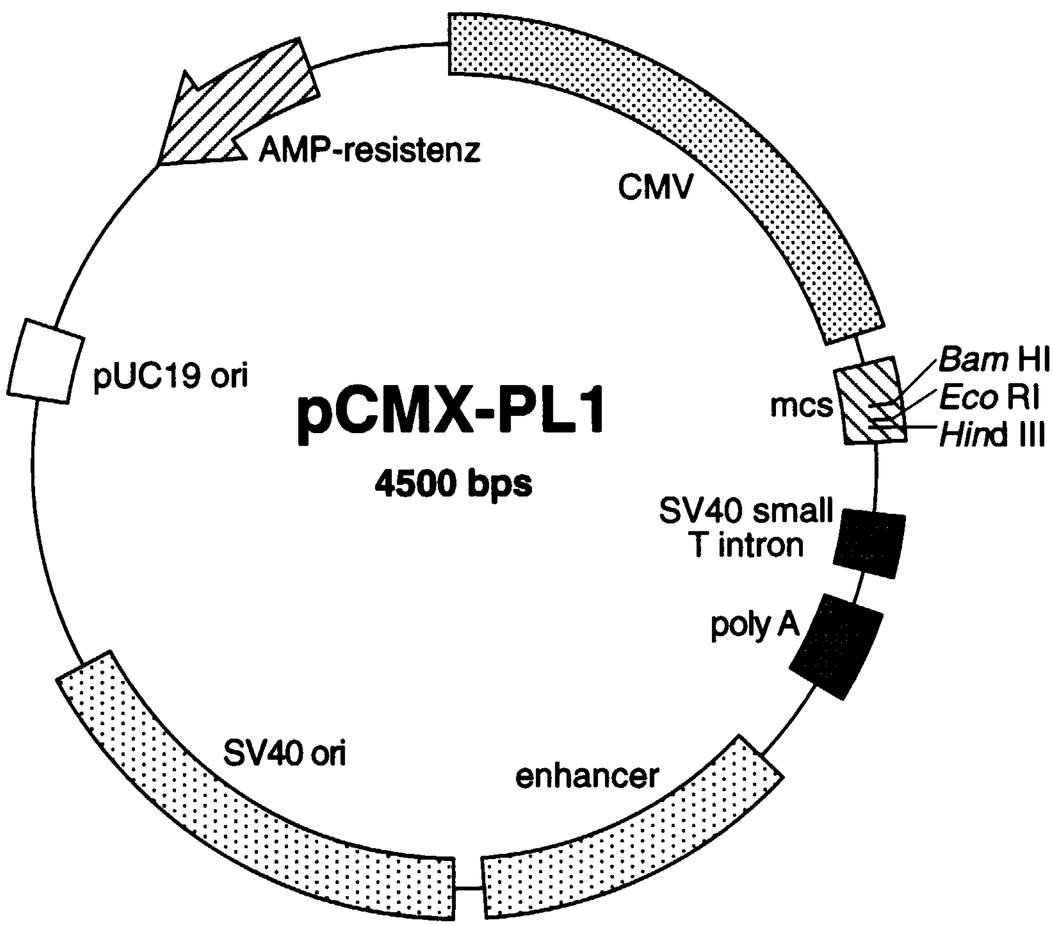
AMP-resistenz
CMV
pUC19 ori
pCMX-PL1
4500 bps
mcs
Bam HI
Eco RI
Hind III
SV40 small
T intron
poly A
SV40 ori
enhancer

SEQUENZPROTOKOLL

(1) ALGEMEINE INFORMATION:

(i) ANMELDER:
(A) NAME: BOEHRINGER MANNHEIM GMBH
(B) STRASSE: Sandhofer Str. 116
(C) ORT: Mannheim
(D) BUNDESLAND: BW
(E) LAND: Germany
(F) POSTLEITZAHL: D-68305
(G) TELEPHON: 08856/60-3446
(H) TELEFAX: 08856/60-3451

(ii) ANMELDETITEL: Melanom-inhibierendes Protein

(iii) ANZAHL DER SEQUENZEN: 24

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(vi) FRÜHERE ANMELDEDATEN:
(A) ANMELDENUMMER: DE P 43 24 247.2
(B) ANMELDEDATUM: 20-JUL-1993

(2) INFORMATION ZU SEQ ID NO: 1:

(i) SEQUENZ CHARAKTERISTIKA:
(A) LÄNGE: 459 Basenpaare
(B) ART: Nukleinsäure
(C) STRANGFORM: Einzel
(D) TOPOLOGIE: linear

(ii) ART DES MOLEKÜLS: cDNS

(ix) MERKMALE:
(A) NAME/SCHLÜSSEL: CDS
(B) LAGE: 40..432

(ix) MERKMALE:
(A) NAME/SCHLÜSSEL: sig_peptide
(B) LAGE: 40..111

(ix) MERKMALE:
(A) NAME/SCHLÜSSEL: mat_peptide
(B) LAGE: 112..432

(xi) SEQUENZBESCHREIBUNG: SEQ ID NO: 1:

```
CCAGCACCCC CTTGCTCACT CTCTTGCTCA CAGTCCACG ATG GCC CGG TCC CTG    54
                                          Met Ala Arg Ser Leu
                                          -24             -20

GTG TGC CTT GGT GTC ATC ATC TTG CTG TCT GCC TTC TCC GGA CCT GGT  102
Val Cys Leu Gly Val Ile Ile Leu Leu Ser Ala Phe Ser Gly Pro Gly
                -15                 -10                 -5

GTC AGG GGT GGT CCT ATG CCC AAG CTG GCT GAC CGG AAG CTG TGT GCG  150
Val Arg Gly Gly Pro Met Pro Lys Leu Ala Asp Arg Lys Leu Cys Ala
              1               5                  10

GAC CAG GAG TGC AGC CAC CCT ATC TCC ATG GCT GTG GCC CTT CAG GAC  198
Asp Gln Glu Cys Ser His Pro Ile Ser Met Ala Val Ala Leu Gln Asp
     15                  20                  25

TAC ATG GCC CCC GAC TGC CGA TTC CTG ACC ATT CAC CGG GGC CAA GTG  246
Tyr Met Ala Pro Asp Cys Arg Phe Leu Thr Ile His Arg Gly Gln Val
 30                  35                  40                  45

GTG TAT GTC TTC TCC AAG CTG AAG GGC CGT GGG CGG CTC TTC TGG GGA  294
Val Tyr Val Phe Ser Lys Leu Lys Gly Arg Gly Arg Leu Phe Trp Gly
                 50                  55                  60

GGC AGC GTT CAG GGA GAT TAC TAT GGA GAT CTG GCT GCT CGC CTG GGC  342
Gly Ser Val Gln Gly Asp Tyr Tyr Gly Asp Leu Ala Ala Arg Leu Gly
             65                  70                  75

TAT TTC CCC AGT AGC ATT GTC CGA GAG GAC CAG ACC CTG AAA CCT GGC  390
Tyr Phe Pro Ser Ser Ile Val Arg Glu Asp Gln Thr Leu Lys Pro Gly
         80                  85                  90

AAA GTC GAT GTG AAG ACA GAC AAA TGG GAT TTC TAC TGC CAG          432
Lys Val Asp Val Lys Thr Asp Lys Trp Asp Phe Tyr Cys Gln
     95                 100                 105

TGAGCTCAGC CTACCGCTGG CCCTGCC                                    459
```

(2) INFORMATION ZU SEQ ID NO: 2:

(i) SEQUENZ CHARAKTERISTIKA:
(A) LÄNGE: 131 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

```
Met Ala Arg Ser Leu Val Cys Leu Gly Val Ile Ile Leu Leu Ser Ala
-24             -20                 -15                 -10

Phe Ser Gly Pro Gly Val Arg Gly Gly Pro Met Pro Lys Leu Ala Asp
             -5                   1               5

Arg Lys Leu Cys Ala Asp Gln Glu Cys Ser His Pro Ile Ser Met Ala
     10                  15                  20

Val Ala Leu Gln Asp Tyr Met Ala Pro Asp Cys Arg Phe Leu Thr Ile
 25                  30                  35                  40

His Arg Gly Gln Val Val Tyr Val Phe Ser Lys Leu Lys Gly Arg Gly
                 45                  50                  55

Arg Leu Phe Trp Gly Gly Ser Val Gln Gly Asp Tyr Tyr Gly Asp Leu
             60                  65                  70

Ala Ala Arg Leu Gly Tyr Phe Pro Ser Ser Ile Val Arg Glu Asp Gln
         75                  80                  85

Thr Leu Lys Pro Gly Lys Val Asp Val Lys Thr Asp Lys Trp Asp Phe
     90                  95                 100

Tyr Cys Gln
105
```

(2) INFORMATION ZU SEQ ID NO: 3:

(i) SEQUENZ CHARAKTERISTIKA:
(A) LÄNGE: 3565 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: sig_peptide
(B) LAGE: 1378..1449

(ix) MERKMALE:
(A) NAME/SCHLÜSSEL: exon
(B) LAGE: 1378..1504

(ix) MERKMALE:
(A) NAME/SCHLÜSSEL: exon
(B) LAGE: 1586..1719

(ix) MERKMALE:
(A) NAME/SCHLÜSSEL: exon
(B) LAGE: 2804..2914

(ix) MERKMALE:
(A) NAME/SCHLÜSSEL: exon
(B) LAGE: 3232..3252

(ix) MERKMALE:
(A) NAME/SCHLÜSSEL: -
(B) LAGE: one-of(2216)
(D) SONSTIGE ANGABEN: /note= "N in Position 2216 steht fuer eine unbestimmte Anzahl und Sequenz von Nukleotiden"

(xi) SEQUENZBESCHREIBUNG: SEQ ID NO: 3:

TCTAGACANA TAAAAATAAA AGAAATCATC CAAGAATGGT GACTTGCCTA CTATTCTACT 60

CGAGAGGCTG AGAGGGGAGG ATTTCTTGAC CCCGGNAGTT TAAGGATGCA GTGAGCTATG 120

ATCACATCAC TGTACTTCAG CCTGAGCAAC AGCAAGATCC TGTCTCTAAA AATTAAATAA 180

GGCTGGGCTT GGTGGCTCAT GCTGTAATCC CAGCACTTTG GAAGGCCATG GTGGGCAGAT 240

TGCTTGAGCC CAGGAGTTTG AGACGAGGCT GGGCAACATG ACGAAACCCC GGCTCTACCA 300

AAAAATACAA AAAATTAACT GGGCATAATG GTACATGTCT GTGGTCCCAG CTACTCGGTA 360

GGCTGAGGTG GGAGGAATGC TTGAGCCCAG GAAATAGGGG CTACAGTGAA CCAGGATGAT 420

GCCAGTGCAC TCCAACCTGG GCAACAGAGC AAGACTCTAC CTCAAAATAA TTTAAAAAAA 480
TGGATTTAATT GGGCATAGGT GGCTTGTGCC TGTAGTCCCA GTTACTCAGG AGCCTGAGGT 540
GGGAGGATTG CCTGAGTCTA GGAGGTTGAG GCTGCAGTGA GCCGGGATGG CACCATTGCA 600
CTCCACCTGG GCAACAGGGT GAGACCCTGT CTCAAAAAAG AAAAAAAAGG GAGGGGTTAT 660
AATCACTCCT CCTGACATGA TACAGAGTAT CCATTTGAGT TCATAACATA AATATGTACT 720
TGGTGAATGC TCTGTAACTA TTGGTGAATG CTCTGTAACT ATTGGCTTTT TTATTGTTCC 780
CATTTTACAT ATAAGGAAGC TGAGGCTTTG TGAGGAGAAA TAGCTTAGCC CAGGTCATCC 840
AGTGGGAAGC GTCTGGTGCA GAGGAATAGT GATCAGGGTG GGACTTTGCC TAGCCTAAGG 900
TTCAGCATAC AATATTCAGT CAGTACTCAA GGGCTGGGCT GTTTCTGGTA ATCAAAGGGC 960
CTGCCTTGTC CTCCTCCCCC ACAGCAGGAA ATTCCAAGGT GGTTTTCTTT ACAGGCTCCT 1020
CCGCTTCTGT GGCCAGAGGG GACAGCGGAG GACCCCAGGT ACCTAAGCCA ACTCAAGAGA 1080
AGATGGAATT GAATATTTCA ACCACCTTAT CTAGGCCTCT GTGATTGTTG AGGAGGGGGC 1140
TGTCACTGGG AAAGTTGTGA GCTGCTTTGG ACCTTATCTG GGAATTTCCT TGGGCCTTAC 1200
AGCTTTACCC TATCCTTGAA ATGGTTCTGG TTTCATAGCA ACTTCTAGGT GGTGTGGGCG 1260
AAGTTTGGGA CTGGTTTAGG GCGGGGACAA GACCAAGAAC ACAAGTTTCC TTGTACGGGA 1320
GAGAGGAAAT TGGAGACCCC AGCACCCCCT TGCTCACTCT CTTGCTCACA GTCCACGATG 1380
GCCCGGTCCC TGGTGTGCCT TGGTGTCATC ATCTTGCTGT CTGCCTTCTC CGGACCTGGT 1440
GTCAGGGGTG GTCCTATGCC CAAGCTGGCT GACCGGAAGC TGTGTGCGGA CCAGGAGTGC 1500
AGCCGTAAGA ATGGGGAGGG GTAGAATTGG GCTTGGGTGT TAGCCTGTGT GGATGTGCTG 1560
CATTCCCCTT CTATTCCTTC CCTAGACCCT ATCTCCATGG CTGTGGCCCT TCAGGACTAC 1620
ATGGCCCCCG ACTGCCGATT CCTGACCATT CACCGGGGCC AAGTGGTGTA TGTCTTCTCC 1680
AAGCTGAAGG GCCGTGGGCG GCTCTTCTGG GGAGGCAGCG TGCGTCTTGG GAGAGTGAAA 1740
GAGGGAAGGG TACAGAGCTG GGGTAGACTC ATTATCCCCA TGAAGGGAAG ATTTGAGGGG 1800

GGTGAACTGA AATAGACATT GTGGGGGGAT ATTGTTACTT ACTTTTATTTT ATTTGCTTAT 1860
TATTTTTTAA TTTTTTCCGA GACAGAGTCT TGCTCTGTCA CCCAGGCTGG ATGCAATGGC 1920
ACGATCTCGG CTCACTGTAA CCTCCACCTC TTGGGTTTAA GCGATTCTCC AGCCTCAGCC 1980
TCCCAAGTAC CTGGGATTAC AGGCATGCAC CACCACACCT NNTAATTTTT GTATTTTTAG 2040
TAGAGACAGG GTTTTACCAT ATTGGCCAGG CTGGTCTTGA ACTCCTGACC TCATGATCTG 2100
CCCGCCTTGG CTCCCGGAGT GCTGGGATTA CAGGTGTGAG CCACTGGCCC CCCAGCCTAT 2160
TTTCACTTTA TTTACCAATT TTAGGACCTG ATATGGTCCC ANNNTCTGTT CTAGANTCTA 2220
GACACCAAGA TACAACAACA AATGATCCTT TTTATTCTAA TGGAGGGAAA TGAACAAAAA 2280
GCAAGGCATA AAAAATAGCA GCAGCCGGGC ACAGTAGCTC ACACCTGTAA TCCCAAGTAA 2340
GGCCAAGTNN GGAGGATAGC TTGAGCCCAG GAGTTCGAGA CCAGCCTGGG CAACATAGCA 2400
AGACCCCCAT CTCTATAAAA AAAAATTTAA AATTAACTGG GCATCATGGC ATGTGTCTGT 2460
GGTCCCGGCT ACTCGGGAGG CTGAGGTGGG AGGATTGCTT GATCCCAGAA GTTGAGGCTG 2520
CAGTGAGCCG TGATCATGCT ACTGCACCTC AACCTGGCCG ACACAATGAG ACCCTGTTTC 2580
CAAAATAATA ATAATAAAAG CAAATATGCG CTGCTGTGAG AATTAACAGA GACTTACTTG 2640
GGTGTTCAGA AAGGGCCTCT GAACAGGTGG CATTTAAGCT GAGATTCATA TGACAAGGAT 2700
GGAGCAGTTA TGTGGAGATC AGGGAGAGGG GAGAATGCAA AGGCCTTCAG CAGGCACAAG 2760
CTTGCCATCT TCCAGACCCT AGCTTTTAAC TCCTCTTCCC CAGGTTCAGG GAGATTACTA 2820
TGGAGATCTG GCTGCTCGCC TGGGCTATTT CCCCAGTAGC ATTGTCCGAG AGGACCAGAC 2880
CCTGAAACCT GGCAAAGTCG ATGTGAAGAC AGACGTGAGT GTCATGGGGG CTGGCAAGAA 2940
ATGTGGGGGG AGGACCCTTA GGTTGTGGGG ATGGGCAAAA ATGCTCCCAC ACTTGGCTCC 3000
CTGGCCGCCT AGGTATGTGC GCTGGGAGAA ATTCTTTCCC TGCCTCAATT TTCTCACCAG 3060
TAAAATGGGT CCAGTTGGGA GGTGCAAAGA TTAGAGGGCT CTAGGCTAAT TTGCATAGCA 3120
NNTGTGTGGC CAGACCTGGG CCCTGCAGCT GCAGCCTTTG CTAAAACCAC TAGATCCTTT 3180

```
GTGGTGTGAC CGCTGGTTTT CTTTCCACTG TTTCCCCTTT CTCTTTTTCA GAAATGGGAT 3240

TTCTACTGCC AGTGAGCTCA GCCTACCGCT GGCCCTGCCG TTTCCCCTCC TTGGGTTTAT 3300

GCAAATACAA TCAGCCCAGT GCAAACGGCT CGTCTCCGTG GTCTTTGGGG TGGGGTAGGG 3360

TAGGGTGGGG ACTGTACAAA TGAAATGTTT CTCTAGGTTG CTGAATCTAA CCAATTAACC 3420

CGCTGCCTGT GGTAACGTCA GTGGTTGCTA GGCAGAGTTT CGCTGATGAA AGCCCTGTGC 3480

AGTAGGAGCG CTCCTAAGCT TAGGTTTCGA CACAAGCAAA GAAAACCTAA GCAGCCCAAC 3540

TAGGGATTGT AGTGTCCTCT CTAGA                                       3565
```

(2) INFORMATION ZU SEQ ID NO: 4:

(i) SEQUENZ CHARAKTERISTIKA:
(A) LÄNGE: 581 Basenpaare
(B) ART: Nukleinsäure
(C) STRANGFORM: Einzel
(D) TOPOLOGIE: linear

(ii) ART DES MOLEKÜLS: cDNS

(ix) MERKMALE:
(A) NAME/SCHLÜSSEL: CDS
(B) LAGE: 110..499

(ix) MERKMALE:
(A) NAME/SCHLÜSSEL: sig_peptide
(B) LAGE: 110..178

(ix) MERKMALE:
(A) NAME/SCHLÜSSEL: mat_peptide
(B) LAGE: 179..499

(xi) SEQUENZBESCHREIBUNG: SEQ ID NO: 4:

```
AAGCGGCGGA GACAGGATCG AGAACACAGG TTTCCTTGAT ATTCAGCCTG GAAGGAGGGC   60

AGGAGGAGCC CAGAGACCTC GTTCTTCACT TGGTCATTCT CAGTCCATG ATG GTG      115
                                                      Met Val
                                                      -23
```

```
TGG TCC CCA GTG CTC CTT GGC ATC GTC GTC TTG TCT GTT TTT TCA GGG    163
Trp Ser Pro Val Leu Leu Gly Ile Val Val Leu Ser Val Phe Ser Gly
    -20                 -15                 -10

CCT AGC AGG GCT GAT CGA GCT ATG CCC AAG CTG GCT GAC TGG AAG CTG    211
Pro Ser Arg Ala Asp Arg Ala Met Pro Lys Leu Ala Asp Trp Lys Leu
 -5                  1                   5                  10

TGT GCG GAC GAG GAA TGC AGC CAT CCT ATC TCC ATG GCT GTG GCC CTC    259
Cys Ala Asp Glu Glu Cys Ser His Pro Ile Ser Met Ala Val Ala Leu
                15                  20                  25

CAG GAC TAC GTG GCC CCT GAT TGC CGC TTC TTG ACT ATA TAT AGG GGC    307
Gln Asp Tyr Val Ala Pro Asp Cys Arg Phe Leu Thr Ile Tyr Arg Gly
            30                  35                  40

CAA GTG GTG TAT GTC TTC TCC AAG TTG AAG GGC CGT GGG CGC CTT TTC    355
Gln Val Val Tyr Val Phe Ser Lys Leu Lys Gly Arg Gly Arg Leu Phe
    45                  50                  55

TGG GGA GGC AGT GTT CAG GGA GGT TAC TAT GGA GAC CTG GCA GCC CGC    403
Trp Gly Gly Ser Val Gln Gly Gly Tyr Tyr Gly Asp Leu Ala Ala Arg
 60                  65                  70                  75

CTG GGC TAT TTC CCC AGT AGC ATT GTC CGG GAG GAC CTG AAC TCG AAA    451
Leu Gly Tyr Phe Pro Ser Ser Ile Val Arg Glu Asp Leu Asn Ser Lys
                    80                  85                  90

CCT GGC AAA ATT GAT ATG AAG ACC GAT CAA TGG GAT TTC TAC TGC CAG    499
Pro Gly Lys Ile Asp Met Lys Thr Asp Gln Trp Asp Phe Tyr Cys Gln
                95                 100                 105

TGAGCTCAGC CTACCGCTAT CCCTGCAGTT ACCTTCCGGC TCTATGCAAA TACAGCAGCC  559

AATGGCAAAA AAAAAAAAAA AA                                           581
```

(2) INFORMATION ZU SEQ ID NO: 5:

(i) SEQUENZ CHARAKTERISTIKA:
(A) LÄNGE: 130 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 5:

```
Met Val Trp Ser Pro Val Leu Leu Gly Ile Val Val Leu Ser Val Phe
-23         -20                 -15                 -10

Ser Gly Pro Ser Arg Ala Asp Arg Ala Met Pro Lys Leu Ala Asp Trp
        -5                  1               5

Lys Leu Cys Ala Asp Glu Glu Cys Ser His Pro Ile Ser Met Ala Val
 10                 15                  20                  25

Ala Leu Gln Asp Tyr Val Ala Pro Asp Cys Arg Phe Leu Thr Ile Tyr
                30                  35                  40

Arg Gly Gln Val Val Tyr Val Phe Ser Lys Leu Lys Gly Arg Gly Arg
            45                  50                  55

Leu Phe Trp Gly Gly Ser Val Gln Gly Gly Tyr Tyr Gly Asp Leu Ala
        60                  65                  70

Ala Arg Leu Gly Tyr Phe Pro Ser Ser Ile Val Arg Glu Asp Leu Asn
    75                  80                  85

Ser Lys Pro Gly Lys Ile Asp Met Lys Thr Asp Gln Trp Asp Phe Tyr
 90                 95                 100                 105

Cys Gln
```

(2) INFORMATION ZU SEQ ID NO: 6:

(i) SEQUENZ CHARAKTERISTIKA:
- (A) LÄNGE: 31 Basenpaare
- (B) ART: Nukleinsäure
- (C) STRANGFORM: Einzel
- (D) TOPOLOGIE: linear

(ii) ART DES MOLEKÜLS: cDNS

(ix) MERKMALE:
- (A) NAME/SCHLÜSSEL: -
- (B) LAGE: one-of(14, 17, 20)
- (D) SONSTIGE ANGABEN: /label= N
  /note= "N steht fuer I (Inosin)"

(xi) SEQUENZBESCHREIBUNG: SEQ ID NO: 6:

TGTGAATTCA GTTTNWSNGCN GAYCARGART G 31

(2) INFORMATION ZU SEQ ID NO: 7:

(i) SEQUENZ CHARAKTERISTIKA:
(A) LÄNGE: 33 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

TGTGTCGACT GTTCGTAGAA RTCCCATCTT RTC 33

(2) INFORMATION ZU SEQ ID NO: 8:

(i) SEQUENZ CHARAKTERISTIKA:
(A) LÄNGE: 305 Basenpaare
(B) ART: Nukleinsäure
(C) STRANGFORM: Einzel
(D) TOPOLOGIE: linear

(ii) ART DES MOLEKÜLS: cDNS

(ix) MERKMALE:
(A) NAME/SCHLÜSSEL: misc_RNA
(B) LAGE: join(1..29, 277..305)
(D) SONSTIGE ANGABEN: /function= "Primer"

(xi) SEQUENZBESCHREIBUNG: SEQ ID NO: 8:

GAATTCAAGT TTTCGGCGGA TCAGGAGTGC AGCCACCCTA TCTCCATGGC TGTGGCCCTT 60

CAGGACTACA TGGCCCCCGA CTGCCGATTC CTGACCATTC ACCGGGGCCA AGTGGTGTAT 120

GTCTTCTCCA AGCTGAAGGG CCGTGGGCGG CTCTTCTGGG GAGGCAGCGT TCAGGGAGAT 180

TACTATGGAG ATCTGGTCGC TCGCCTGGGC TATTTCCCCA GTAGCATTGT CCGAGAGGAC 240

CAGACCCTGA AACCTGGCAA AGTCGATGTG AAGACAGATA AATGGGATTT CTACGAACAG 300

TCGAC 305

(2) INFORMATION ZU SEQ ID NO: 9:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 9:

GATGCATGCG GTCCTATGCC CAAGCTG 27

(2) INFORMATION ZU SEQ ID NO: 10:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 10:

GATAAGCTTT CACTGGCAGT AGAAATC 27

(2) INFORMATION ZU SEQ ID NO: 11:

(i) SEQUENZ CHARAKTERISTIKA:
(A) LÄNGE: 28 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 11:

GATCTAGCCG GCCGCCCAGC CCGGCATG 28

(2) INFORMATION ZU SEQ ID NO: 12:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 12:

CCGGGCTGGG CGGCCGGCTA 20

(2) INFORMATION ZU SEQ ID NO: 13:

(i) SEQUENZ CHARAKTERISTIKA:
(A) LÄNGE: 16 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 13:

```
Met Arg Gly Ser His His His His His His Gly Ser Ser Arg Pro Pro
1               5                   10                  15
```

(2) INFORMATION ZU SEQ ID NO: 14:

(i) SEQUENZ CHARAKTERISTIKA:
(A) LÄNGE: 18 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 14:

```
Met Arg Gly Ser His His His His His His Gly Ser Val Asp Asp Asp
1               5                   10                  15

Asp Lys
```

(2) INFORMATION ZU SEQ ID NO: 15:

(i) SEQUENZ CHARAKTERISTIKA:
(A) LÄNGE: 43 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 15:

```
AAAAAGGATC CAGCCGGCCG CCCGGTCCTA TGCCCAAGCT GGC                   43
```

(2) INFORMATION ZU SEQ ID NO: 16:

(i) SEQUENZ CHARAKTERISTIKA:
(A) LÄNGE: 23 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 16:

GGCGAGCAGC CAGATCTCCA TAG 23

(2) INFORMATION ZU SEQ ID NO: 17:

(i) SEQUENZ CHARAKTERISTIKA:
(A) LÄNGE: 50 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 17:

AAAAAAGGAT CCGTTGATGA TGACGATAAA GGTCCTATGC CCAAGCTGGC 50

(2) INFORMATION ZU SEQ ID NO: 18:

(i) SEQUENZ CHARAKTERISTIKA:
(A) LÄNGE: 330 Basenpaare
(B) ART: Nukleinsäure
(C) STRANGFORM: Einzel
(D) TOPOLOGIE: linear

(ii) ART DES MOLEKÜLS: cDNS

(ix) MERKMALE:
(A) NAME/SCHLÜSSEL: mat_peptide
(B) LAGE: 7..327

(ix) MERKMALE:
(A) NAME/SCHLÜSSEL: misc_RNA
(B) LAGE: 4..6
(D) SONSTIGE ANGABEN: /function= "Startcodon Met"

(xi) SEQUENZBESCHREIBUNG: SEQ ID NO: 18:

```
CATATGGGAC CAATGCCAAA ATTAGCAGAT CGTAAATTAT GTGCAGATCA GGAGTGCAGC   60
CACCCTATCT CCATGGCTGT GGCCCTTCAG GACTACATGG CCCCCGACTG CCGATTCCTG  120
ACCATTCACC GGGGCCAAGT GGTGTATGTC TTCTCCAAGC TGAAGGGCCG TGGGCGGCTC  180
TTCTGGGGAG GCAGCGTTCA GGGAGATTAC TATGGAGATC TGGCTGCTCG CCTGGGCTAT  240
TTCCCCAGTA GCATTGTCCG AGAGGACCAG ACCCTGAAAC CTGGCAAAGT CGATGTGAAG  300
ACAGACAAAT GGGATTTCTA CTGCCAGTGA                                    330
```

(2) INFORMATION ZU SEQ ID NO: 19:

(i) SEQUENZ CHARAKTERISTIKA:
(A) LÄNGE: 59 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 19:

```
AAAAACATAT GGGACCAATG CCAAAATTAG CAGATCGTAA ATTATGTGCA GATCAGGAG   59
```

(2) INFORMATION ZU SEQ ID NO: 20:

(i) SEQUENZ CHARAKTERISTIKA:
(A) LÄNGE: 29 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 20:

AAAAAAAGCT TTCACTGGCA GTAGAAATC 29

(2) INFORMATION ZU SEQ ID NO: 21:

(i) SEQUENZ CHARAKTERISTIKA:
(A) LÄNGE: 33 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 21:

CGTGAATTCA ACATGGCCCG GTCCCTGGTG TGC 33

(2) INFORMATION ZU SEQ ID NO: 22:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 22:

TATCTGCAGT CACTGGCAGT AGAAATCCCA 30

(2) INFORMATION ZU SEQ ID NO: 23:

(i) SEQUENZ CHARAKTERISTIKA:
(A) LÄNGE: 260 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 23:

GTGGGAGGTC TATATAAGCA GAGCTCTCTG GCTAACTAGA GAACCCACTG CTTAACTGGC 60

TTATCGAAAT TAATACGACT CACTATAGGG AGACCCAAGC TGTACCAGAT ATCAGGATCC 120

CCCGGGCTGC AGGAATTCGA TATCAAGCTT CTCGAGGGGG GGCCCGGTAC CGATCCTGGC 180

CAGCTAGCTA GTAGCTAGAG GATCTTTGTG AAGGAACCTT ACTTCTGTGG TGTGACATAA 240

TTGGACAAAC TACCTACAGA 260

(2) INFORMATION ZU SEQ ID NO: 24:

(i) SEQUENZ CHARAKTERISTIKA:
(A) LÄNGE: 596 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: CDS
(B) LAGE: join(40..111, 40..166, 214..347, 393..503, 549 ..569)

(ix) MERKMALE:
(A) NAME/SCHLÜSSEL: sig_peptide
(B) LAGE: 40..111

(ix) MERKMALE:
(A) NAME/SCHLÜSSEL: exon
(B) LAGE: 40..166

(ix) MERKMALE:
(A) NAME/SCHLÜSSEL: exon
(B) LAGE: 214..347

(ix) MERKMALE:
(A) NAME/SCHLÜSSEL: exon
(B) LAGE: 393..503

(ix) MERKMALE:
(A) NAME/SCHLÜSSEL: exon
(B) LAGE: 549..569

(ix) MERKMALE:
(A) NAME/SCHLÜSSEL: -
(B) LAGE: one-of(194, 369, 527)
(D) SONSTIGE ANGABEN: /note= "N in den Positionen 194, 369 und 527 steht fuer eine unbestimmte Anzahl und Sequenz von Nukleotiden "

(xi) SEQUENZBESCHREIBUNG: SEQ ID NO: 24:

```
CCAGCACCCC CTTGCTCACT CTCTTGCTCA CAGTCCACGA TGGCCCGGTC CCTGGTGTGC  60
CTTGGTGTCA TCATCTTGCT GTCTGCCTTC TCCGGACCTG GTGTCAGGGG TGGTCCTATG 120
CCCAAGCTGG CTGACCGGAA GCTGTGTGCG GACCAGGAGT GCAGCCGTAA GAATGGGGAG 180
GGTAGAATTG GGNCCCTTCT ATTCCTTCCC TAGACCCTAT CTCCATGGCT GTGGCCCTTC 240
AGGACTACAT GGCCCCCGAC TGCCGATTCC TGACCATTCA CCGGGGCCAA GTGGTGTATG 300
TCTTCTCCAA GCTGAAGGGC CGTGGGCGGC TCTTCTGGGG AGGCAGCGTG GGTCTTGGGA 360
GAGTGAAANA GCTTTTAACT CCTCTTCCCC AGGTTCAGGG AGATTACTAT GGAGATCTGG 420
CTGCTCGCCT GGGCTATTTC CCCAGTAGCA TTGTCCGAGA GGACCAGACC CTGAAACCTG 480
GCAAAGTCGA TGTGAAGACA GACGTGGAGT GTCATGGGGG CTGGCANTTT CCCCTTTCTC 540
TTTTTCAGAA ATGGGATTTC TACTGCCAGT GAGCTCAGCC TACCGCTGGC CCTGCC     596
```